(19) Europäisches Patentamt / European Patent Office / Office européen des brevets

(11) **EP 2 716 230 A1**

(12) **EUROPEAN PATENT APPLICATION**
published in accordance with Art. 153(4) EPC

(43) Date of publication:
**09.04.2014 Bulletin 2014/15**

(51) Int Cl.:
**A61B 8/00** (2006.01)

(21) Application number: **12792497.5**

(22) Date of filing: **30.05.2012**

(86) International application number:
**PCT/JP2012/003523**

(87) International publication number:
**WO 2012/164919 (06.12.2012 Gazette 2012/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2011  JP 2011119986**

(71) Applicant: **Panasonic Corporation Osaka 571-8501 (JP)**

(72) Inventors:
• **TOMA, Tadamasa**
  **Chuo-ku**
  **Osaka-shi, Osaka 540-6207 (JP)**
• **OHMIYA, Jun**
  **Chuo-ku**
  **Osaka-shi, Osaka 540-6207 (JP)**
• **TOJI, Bumpei**
  **Chuo-ku**
  **Osaka-shi, Osaka 540-6207 (JP)**

(74) Representative: **Vigand, Philippe et al**
  **Novagraaf International SA**
  **3 chemin de l'Echo**
  **1213 Onex Geneva (CH)**

(54) **ULTRASOUND IMAGE-GENERATING APPARATUS AND ULTRASOUND IMAGE-GENERATING METHOD**

(57)    An ultrasound image generation apparatus (10) includes: a first motion estimation unit (10A) which estimates, as a first motion vector, a motion vector of the ultrasound probe using a first estimation method, the motion vector indicating the movement between images of the respective ultrasound signals; a second motion estimation unit (10B) which estimates, as a second motion vector, a motion vector of the ultrasound probe using a second estimation method different from the first estimation method in direction dependency of estimation accuracy; and a position reconstruction unit (10C) which (i) assigns, based on a direction of the first motion vector or a direction of the second motion vector, weights to the first and the second motion vectors, (ii) combines the weighted first and second motion vectors into a combined motion vector, and (iii) constructs an ultrasound diagnostic image of a subject using the combined motion vector and the images.

FIG. 1A

EP 2 716 230 A1

FIG. 1B

**Description**

[Technical Field]

The present invention relates to ultrasound image generation apparatuses and ultrasound image generation methods. The present invention particularly relates to an ultrasound image generation apparatus and an ultrasound image generation method by which an ultrasound diagnostic image is generated from a plurality of ultrasound signals received from a subject while an ultrasound probe is being moved.

[Background Art]

An x-ray diagnostic apparatus, a magnetic resonance (MR) diagnostic apparatus, or an ultrasonic diagnostic apparatus is widely used as an image diagnostic apparatus for a living body. The ultrasonic diagnostic apparatus is useful for its noninvasive and real-time nature, and is used to make a medical examination and other various diagnoses. Various sites are diagnosed using the ultrasonic diagnostic apparatus, such as the heart, a blood vessel such as a carotid artery, the liver, or a breast. Among these, the carotid artery is one of the crucial sites of diagnosis because recent years have seen an increase in the number of individual affected by arteriosclerosis.

The following describes the carotid artery as an example. In the diagnosis of the carotid artery using the ultrasound, observation is made along the common carotid artery to the internal carotid artery or to the external carotid artery to examine, for example, the intima-media thickness (IMT) or whether or not plaque is present. At this time, it is necessary to scan the ultrasound probe in more than one direction to fully observe the interior of a blood vessel. However, the above-described method has problems. For example, the inspection is time-consuming because the blood vessel configuration is hard to find, or it is difficult to ensure reproducibility because the result of observation depends on the skill of the examiner. The need for a diagnosis based on a three-dimensional image of a blood vessel constructed from the scanned image has been growing to solve the above described problem.

When constructing a three-dimensional image from the ultrasound images obtained by the scanning, position information (position and orientation) of the ultrasound probe at the time of obtainment of the ultrasound images is obtained, and each of the ultrasound images are mapped to a three-dimensional space based on the position information. For example, the position information is estimated by capturing, with a camera, an image of a marker attached to the ultrasound probe. The position of the ultrasound probe is estimated based on the change in the position and shape of the marker in the captured image (e.g., patent literature (PTL) 1).

[Citation List]

[Patent Literature]

[PTL 1]

Japanese Unexamined Patent Application Publication No. 2010-75503

[Summary of Invention]

[Technical Problem]

However, there is a problem that the position obtainment accuracy of the ultrasound probe is directionally dependent.

The present invention has been conceived in view of the above problem, and has an object to provide an ultrasound image generation apparatus which reduces direction dependency of accuracy in obtaining the position of the ultrasound probe.

[Solution to Problem]

In order to achieve the aforementioned object, an ultrasound image generation apparatus according to an aspect of the present invention is an ultrasound image generation apparatus which generates an ultrasound diagnostic image from a plurality of ultrasound signals received from a subject while an ultrasound probe is being moved, the ultrasound image generation apparatus includes: a first motion estimation unit configured to estimate, as a first motion vector, a motion vector indicating a movement of the ultrasound probe using a first estimation method, the motion vector indicating the movement between a plurality of images of the respective ultrasound signals; a second motion estimation unit configured to estimate, as a second motion vector, a motion vector indicating a movement of the ultrasound probe using a second estimation method which is different from the first estimation method in direction dependency of estimation accuracy, the motion vector indicating the movement between the images of the respective ultrasound signals; and a position reconstruction unit configured to (i) assign, based on a direction of the first motion vector or a direction of the second motion vector, weights to the first motion vector estimated by the first motion estimation unit and the second motion vector estimated by the second motion estimation unit, (ii) combine the weighted first and second motion vectors into a combined motion vector, and (iii) construct the ultrasound diagnostic image of the subject using the combined motion vector and the images.

It should be noted that these general and specific aspects may be implemented using a system, a method, an integrated circuit, a computer program, or a computer-readable recording medium such as a CD-ROM, or any combination of systems, methods, integrated circuits, computer programs, or computer-readable recording media.

[Advantageous Effects of Invention]

The ultrasound image generation apparatus according to an aspect of the present invention makes it possible to obtain position information with high accuracy in regard to movement of the ultrasound probe in any direction, and construct a three-dimensional image more accurately than a three-dimensional image constructed by a conventional ultrasound image generation apparatus.

[Brief Description of Drawings]

[FIG. 1A] FIG. 1A is a configuration diagram of an example of an ultrasound image generation apparatus according to Embodiment 1.
[FIG. 1B] FIG. 1B is a configuration diagram of another example of the ultrasound image generation apparatus according to Embodiment 1.
[FIG. 2] FIG. 2 is a descriptive diagram of a method for obtaining a motion vector by image processing.
[FIG. 3] FIG. 3 is a flowchart of operations performed by the ultrasound image generation apparatus according to Embodiment 1.
[FIG. 4] FIG. 4 is a diagram showing a configuration of a position reconstruction unit.
[FIG. 5] FIG. 5 is a descriptive diagram of an angle formed by a reference direction and a motion vector.
[FIG. 6] FIG. 6 is a flowchart of operations performed by the position reconstruction unit.
[FIG. 7] FIG. 7 is a diagram showing a method for resolving the motion vector into a reference direction component and an orthogonal component.
[FIG. 8] FIG. 8 is a diagram showing a method for resolving the motion vector into components of an absolute coordinate space.
[FIG. 9] FIG. 9 is a descriptive diagram of a method for obtaining position information by a plurality of cameras.
[FIG. 10] FIG. 10 is a diagram showing advantageous effects of the ultrasound image generation apparatus according to Embodiment 1.
[FIG. 11] FIG. 11 is a diagram showing advantageous effects of an ultrasound image generation apparatus according to Embodiment 2.
[FIG. 12] FIG. 12 is a flowchart of operations performed by the ultrasound image generation apparatus according to Embodiment 2.
[FIG. 13] FIG. 13 is a descriptive diagram on arrangement of cameras, and assist information for arranging cameras.
[FIG. 14A] FIG. 14A is a flowchart of operations performed by a variation of the ultrasound image generation apparatus according to Embodiment 2.
[FIG. 14B] FIG. 14B is a configuration diagram of an example of the ultrasound image generation apparatus including a feedback unit.
[FIG. 15] FIG. 15 is a configuration diagram of an example of a conventional ultrasound image generation apparatus.
[FIG. 16] FIG. 16 is a flowchart of operations performed by the conventional ultrasound image generation apparatus.
[FIG. 17] FIG. 17 is a descriptive diagram of a method for obtaining a position performed by the conventional ultrasound image generation apparatus.
[FIG. 18] FIG. 18 is a descriptive diagram which describes a case in which the ultrasound image generation method is implemented by a computer system with a program recorded on a recoding medium such as a flexible disk.

[Description of Embodiments]

(Underlying knowledge forming basis of the present invention)

The inventors found that the ultrasonic diagnostic apparatus described in the section of "Background Art" causes the following problems.

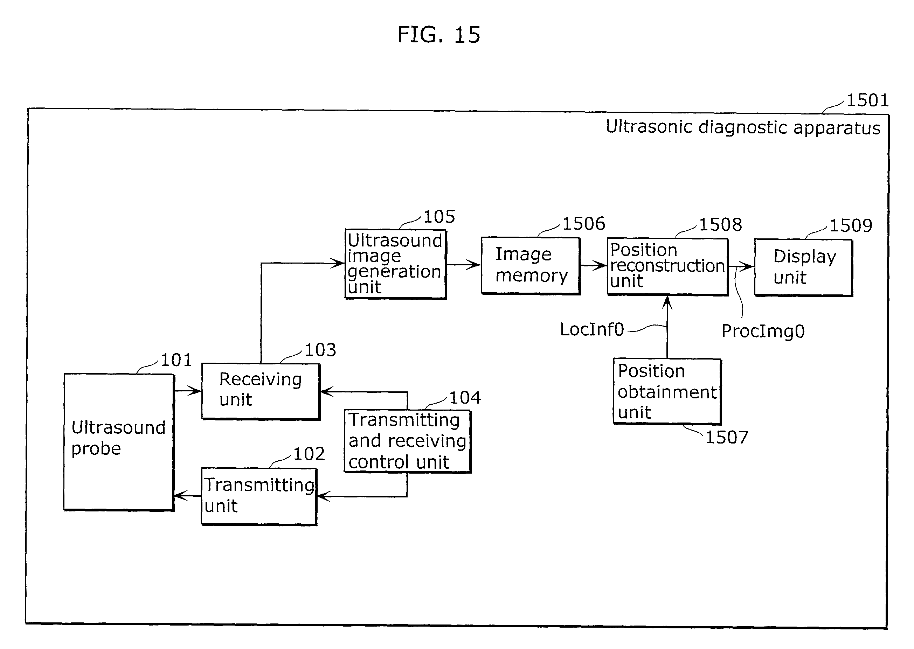

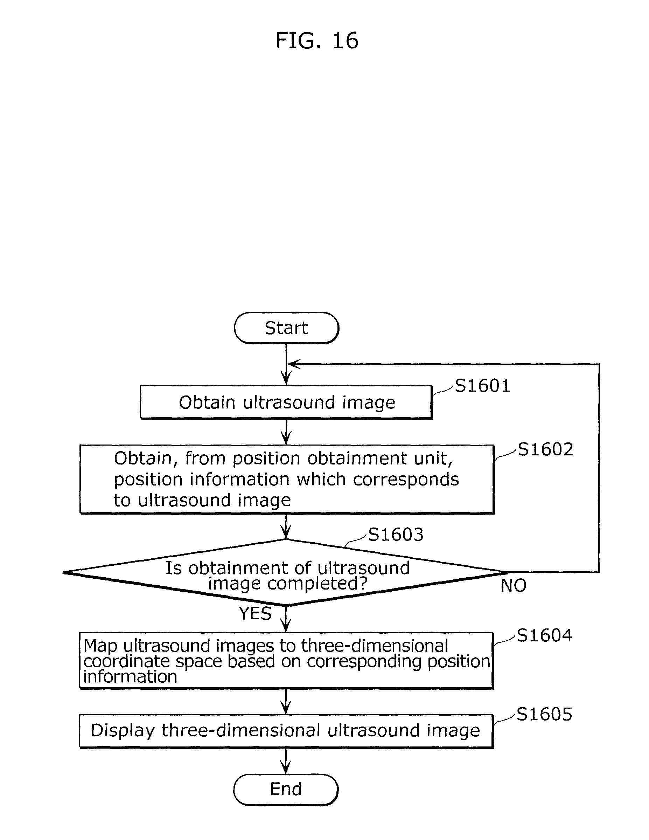

A conventional ultrasonic diagnostic apparatus is described with reference to FIG. 15 and FIG. 16, which constructs a three-dimensional image based on position information of an ultrasound probe obtained at the time of the scan.

FIG. 15 is a configuration diagram of an example of a conventional ultrasound image generation apparatus 1501. As shown in FIG. 15, the ultrasonic diagnostic apparatus 1501 includes: an ultrasound probe 101, a transmitting unit 102, a receiving unit 103, a transmitting and receiving control unit 104, an ultrasound image generation unit 105, an image memory 1506, a position obtainment unit 1507, a position reconstruction unit 1508, and a display unit 1509.

The element, such as a piezoelectric element, disposed in the ultrasound probe 101 generates an ultrasound signal based on a drive signal output from the transmitting unit 102. The ultrasound signal is reflected by the structure, such as a blood vessel wall and a muscle, inside the living body of a subject, and part of the reflection component is received by the ultrasound probe 101.

The receiving unit 103 sequentially performs, on the received reflection signal, amplification, A/D (analog-to-digital) conversion, delay adding processing of a signal of each element, and the like to generate a receipt radio frequency (RF) signal.

The transmitting and receiving control unit 104 controls operations of the transmitting unit 102, and the receiving unit 103. For the transmitting unit 102, for example, the transmitting and receiving control unit 104 switches driving voltages, and sets transmission frequency. For the receiving unit 103, for example, the transmitting and receiving control unit 104 sets delay time to allow reception beamforming to be performed.

The ultrasound image generation unit 105 converts the receipt RF signal into an ultrasound image, and stores the ultrasound image in the image memory 1506. Examples of the generated ultrasound image are: a B-mode image which represents the signal strength by the intensity of brightness; and a Doppler image which shows bloodstream and the speed of movement of tissue which are calculated based on the Doppler effect of the receipt RF signal.

The position obtainment unit 1507 obtains position information LocInf0 of the ultrasound probe, and outputs the position information LocInf0 to the position reconstruction unit 1508. The position obtainment unit 1507 is, for example, implemented by camera.

Based on the position information LocInf0 received from the position obtainment unit 1507, the position reconstruction unit 1508 maps the ultrasound images, which are stored in the image memory 1506, to a three-dimensional space to construct a three-dimensional image ProcImg0 of the imaging target.

The display unit 1509 displays the three-dimensional image ProcImg0 on a display device, such as a monitor.

FIG. 16 is a flowchart showing operations performed by the conventional ultrasound image generation apparatus 1501.

The conventional ultrasound image generation apparatus 1501 obtains the ultrasound image in Step S1601. In Step S1602, the ultrasound image generation apparatus 1501 obtains, from the position obtainment unit 1507, position information which corresponds to the ultrasound image obtained in Step S1601. In Step S1603, the ultrasound image generation apparatus 1501 determines whether or not the obtainment of the ultrasound image has been completed. The ultrasound image generation apparatus 1501 repeats the processing in Step S1601 and Step S1602 until it is determined in Step 1603 that the obtainment of the ultrasound image has been completed. In Step S1604, the ultrasound image generation apparatus 1501 maps the obtained ultrasound images to the three-dimensional space based on the position information. In Step S1605, the ultrasound image generation apparatus 1501 displays the three-dimensional ultrasound image.

High-accuracy three-dimensional images are essential for increasing the accuracy of the diagnosis which is made based on the three-dimensional image. It is necessary to accurately obtain position information to obtain the high-accuracy three-dimensional image. However, the conventional ultrasonic diagnostic apparatus determines the position information based on the information obtained by a single position obtainment unit. When the position obtainment unit has direction dependency of accuracy in obtaining the position information, there is a problem that the accuracy of the position information decreases in the direction in which the position obtainment accuracy is low, and the accuracy in constructing the three-dimensional image decreases.

The position information can be obtained using a position sensor, such as a camera; or by image processing using ultrasound images. The following describes the direction dependency of position resolution, with examples of position estimation using the camera and the image processing.

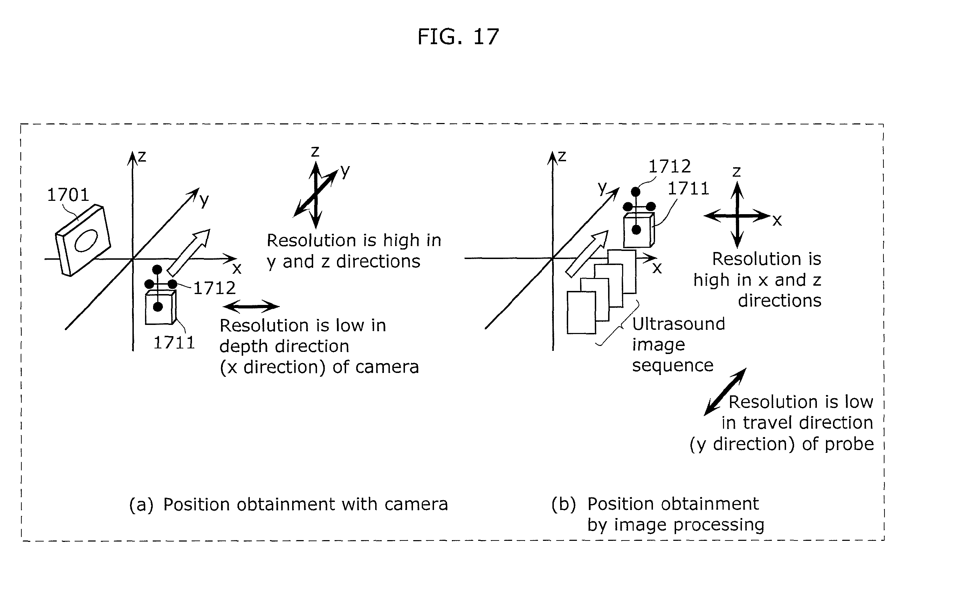

FIG. 17 is a descriptive diagram of a method for obtaining position information performed by the conventional ultrasound image generation apparatus.

Shown in (a) in FIG. 17 is a descriptive diagram of a method for obtaining position information with the camera. The camera is disposed toward x-axis direction, tracks an optical marker (marker) attached to the ultrasound probe, and determines the position information of the marker based on the amount of change in the shape or the orientation of the marker. Assuming that the marker moves the same distance, the amount of change in the shape or the orientation of the marker is different depending on the direction in which the marker moves. The amount of change is smaller when the marker moves in the x-direction compared to the case in which the marker moves in the y-axis direction or the z-axis direction. The resolution of the position information is low when the amount of change is small. Therefore, when the position information is obtained using a camera, the resolution in the x-axis direction that is the depth direction of the camera is low.

Shown in (b) in FIG. 17 is a descriptive diagram of a method for obtaining position information by the image processing. In this method, the amount of displacement in position between the ultrasound images which are continuously scanned is determined based on the correlation or the like between the images to determine the relative position relationship between the images. For example, by finding the amount of displacement in position between two consecutive B-mode images which are (N - 1)-th image and N-th image, the motion vector between (N - 1)-th image and N-th image can be determined. However, according to the method using the correlation between images, the resolution in the travelling direction (corresponding to the y-axis direction) of the probe is low compared to the amount of displacement on the image plane (a plane formed by the x-axis and the z-axis).

As described, the position information obtained by a single technique has direction dependency. Thus, when images are captured while the probe is being moved in arbitrary directions, a direction exists in which the resolution of the position information is low. When the three-dimensional image is constructed from the ultrasound images, the ultrasound images are mapped to the three-dimensional space based on the position information. Thus, there is a problem that the low resolution position information leads to decreased accuracy in constructing the three-dimensional image.

The present invention has been conceived in view of the above problem, and has an object to provide an ultrasound image generation apparatus which reduces direction dependency of accuracy in obtaining the position of the ultrasound probe.

In order to solve the aforementioned problem, an ultrasound image generation apparatus according to an aspect of the present invention is an ultrasound image generation apparatus which generates an ultrasound diagnostic image from a plurality of ultrasound signals received from a subject while an ultrasound probe is being moved, the ultrasound image generation apparatus includes: a first motion estimation unit configured to estimate, as a first motion vector, a motion vector indicating a movement of the ultrasound probe using a first estimation method, the motion vector indicating the movement between a plurality of images of the respective ultrasound signals; a second motion estimation unit configured to estimate, as a second motion vector, a motion vector indicating a movement of the ultrasound probe using a second estimation method which is different from the first estimation method in direction dependency of estimation accuracy, the motion vector indicating the movement between the images of the respective ultrasound signals; and a position reconstruction unit configured to (i) assign, based on a direction of the first motion vector or a direction of the second motion vector, weights to the first motion vector estimated by the first motion estimation unit and the second motion vector estimated by the second motion estimation unit, (ii) combine the weighted first and second motion vectors into a combined motion vector, and (iii) construct the ultrasound diagnostic image of the subject using the combined motion vector and the images.

With this, a plurality of the motion vectors are estimated using the first and the second estimation methods having different direction dependency of the estimation accuracy, and the estimated motion vectors are combined. Thus, it is possible to obtain the motion vector of the ultrasound probe by combining the component of a direction in which the estimation accuracy from the first estimation method is relatively low with the component of a direction in which the estimation accuracy from the second estimation method is relatively high. At this time, it is possible to calculate the motion vector having high accuracy in all directions by assigning a lighter weight to the component having lower estimation accuracy and assigning a heavier weight to the component having higher estimation accuracy. Thus, it is possible to reduce direction dependency of accuracy in obtaining the position of the ultrasound probe.

Furthermore, for example, the first motion estimation unit is configured to estimate a motion vector of the ultrasound probe by calculating, based on image displacement between two images among the images, the motion vector indicating a movement made from when one of the two images is obtained till when the other of the two images is obtained.

With this, it is possible to analyze the displacement of the two images and estimate the amount of movement of the ultrasound probe. This makes it possible to detect, with relatively high accuracy, the motion of the ultrasound probe on a plane including the cross-section of the subject which transmitted the ultrasound signals corresponding to the images.

Furthermore, for example, the first motion estimation unit is configured to estimate the motion vector of the ultrasound probe from when the one of the two images among the images is obtained till when the other of the two images is obtained, by detecting an amount of the image displacement between the two images based on a correlation between pixel values of pixels included in the two images.

With this, it is possible to analyze the pixels which are included in the two images to calculate the amount of movement of the corresponding pixel or region, which is formed by a plurality of pixels, and estimate the amount of movement of the ultrasound probe based on the calculated amount of movement. This makes it possible to detect, with relatively high accuracy, the motion of the ultrasound probe on a plane including the cross-section of the subject corresponding to the images.

Furthermore, for example, the position reconstruction unit is configured to (i) calculate the combined motion vector by assigning a heavier weight to the first motion vector or the second motion vector when an angle formed by a movement direction of the ultrasound probe and a corresponding one of reference directions is greater, and (ii) construct the ultrasound diagnostic image using the combined motion vector, the corresponding one of the reference directions being a direction used as a reference by the first motion estimation unit or the second motion estimation unit when the estimation is made.

With this, when generating the combined vector, a heavier weight is set to the component of a direction in which the estimation accuracy is relatively high from the estimation method, and then the weight is assigned to the estimated position vector. This makes it possible to increase the accuracy of the combined motion vector. Thus, it is possible to reduce direction dependency of accuracy in obtaining the position of the ultrasound probe.

Furthermore, for example, the position reconstruction unit is configured to (i) calculate the combined motion vector by assigning a heavier weight to the motion vector estimated by the first motion estimation unit when an angle formed by a movement direction of the ultrasound probe and the reference direction used by the first motion estimation unit is greater, and (ii) construct the ultrasound diagnostic image using the combined motion vector, the reference direction being a direction in which estimation accuracy is low.

With this, when estimating the motion vector of the ultrasound probe by image analysis, a heavier weight is set to the direction component on a plane including the cross-section of the subject corresponding to the images, and then the combined position vector is generated. This makes it possible to increase the accuracy of the combined motion vector.

Furthermore, for example, at least one of the first motion estimation unit and the second motion estimation unit is configured to capture, with a camera, an image of an optical marker attached to the ultrasound probe, and calculate a position and an angle of the ultrasound probe based on a position or a shape of the optical marker in the captured image.

With this, it is possible to estimate the motion vector of the ultrasound probe by the optical marker and the camera. This makes it possible to detect, with relatively high accuracy, the motion of the ultrasound probe on a plane parallel to the imaging surface of the camera.

Furthermore, for example, the position reconstruction unit is configured to (i) calculate the combined motion vector by assigning a heavier weight to the motion vector estimated by the optical motion estimation unit when an angle formed by a movement direction of the ultrasound probe and a normal direction of an imaging surface of the camera is greater, and (ii) assemble the images using the combined motion vector, the normal direction being a reference direction used by the optical motion estimation unit.

With this, when estimating the ultrasound probe by the optical marker and camera, a heavier weight is set to the direction component on a plane parallel to the imaging surface of the camera, and then the combined position vector is generated. This makes it possible to increase the accuracy of the combined motion vector.

Furthermore, for example, the first motion estimation unit is configured to estimate a motion vector of the ultrasound probe by calculating, based on image displacement between two images among the images, the motion vector indicating a movement made from when one of the two images is obtained till when the other of the two images is obtained, and the second motion estimation unit is configured to estimate a motion vector of the ultrasound probe by capturing, with a camera, an image of an optical marker attached to the ultrasound probe, and calculating a position and an angle of the ultrasound probe based on a position or a shape of the optical marker in the captured image.

With this, it is possible to use both methods, namely, the position estimation of the ultrasound probe by the image processing, and the position estimation of the ultrasound probe by the optical marker and camera, and generate the combined position vector by combining the position vectors of the ultrasound probe which are estimated using the aforementioned methods.

Furthermore, for example, the ultrasound image generation apparatus further includes a placement assist unit configured to present information which prompts an operator to perform an operation for changing at least one of (i) a reference direction used by the first motion estimation unit and (ii) a reference direction used by the second motion estimation unit to allow the reference directions to be substantially orthogonal to each other.

With this, an operator can set the direction of the estimation method so that the direction in which the estimation accuracy from an estimation method is low matches the direction in which the estimation accuracy is high from another estimation method, in order to reduce the direction dependency of accuracy in estimating the combined position vector. Thus, it is possible to reduce direction dependency of accuracy in obtaining the position of the ultrasound probe.

Furthermore, an ultrasound image generation method according to an aspect of the present invention is an ultrasound image generation method for generating an ultrasound diagnostic image from a plurality of ultrasound signals received from a subject while an ultrasound probe is being moved, the ultrasound image generation method includes: estimating, as a first motion vector, a motion vector indicating a movement of the ultrasound probe using a first estimation method, the motion vector indicating the movement between a plurality of images of the respective ultrasound signals; estimating, as a second motion vector, a motion vector indicating a movement of the ultrasound probe using a second estimation method which is different from the first estimation method in direction dependency of estimation accuracy, the motion vector indicating the movement between the images of the respective ultrasound signals; and (i) assigning, based on a direction of the first motion vector or a direction of the second motion vector, weights to the first motion vector estimated in the estimating of the first motion vector and the second motion vector estimated in the estimating of the second motion vector, (ii) combining the weighted first and second motion vectors into a combined motion vector, and (iii) constructing the ultrasound diagnostic image of the subject using the combined motion vector and the images.

This produces advantageous effects similar to the advantageous effects produced by the above-described ultrasound image generation apparatus.

Furthermore, in the ultrasound image generation method according to an aspect of the present invention, in the estimating of the first motion vector, a motion vector of the ultrasound probe is estimated by calculating, based on image displacement between two images among the images, the motion vector indicating a movement made from when one of the two images is obtained till when the other of the two images is obtained.

This produces advantageous effects similar to the advantageous effects produced by the above-described ultrasound image generation apparatus.

The following describes the ultrasound image generation apparatus according to an aspect of the present invention with reference to the drawings.

It should be noted that each of the embodiments described hereafter illustrates a specific example of the present invention. Numerical values, shapes, materials, constituent elements, the positioning and connection configuration of the constituent elements, steps, the sequence of the steps, and so on, described in the embodiments below are merely examples and are not intended to limit the present invention. Furthermore, among the constituent elements in the following embodiments, those constituent elements which are not described in the independent claims indicating the broadest concept of the present invention are described as optional constituent elements.

(Embodiment 1)

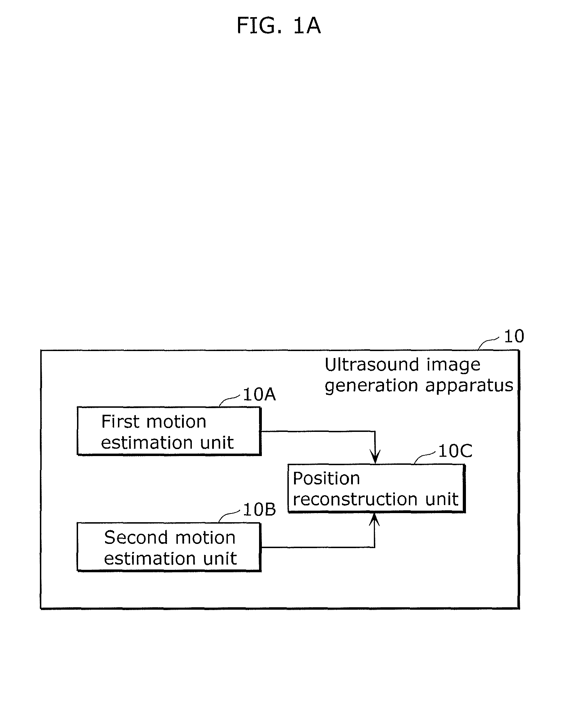

FIG. 1A is a configuration diagram of an ultrasound image generation apparatus 10 according to Embodiment 1 of the present invention.

As FIG. 1A shows, the ultrasound image generation apparatus 10 according to Embodiment 1 of the present invention includes: a first motion estimation unit 10A, a second motion estimation unit 10B, and a position reconstruction unit 10C.

The first motion estimation unit 10A estimates a motion vector of the ultrasound probe using a first estimation method. The motion vector indicates movement between a plurality of images of the respective ultrasound signals.

The second motion estimation unit 10B estimates a motion vector of the ultrasound probe using a second estimation method which is different from the first estimation method in direction dependency of estimation accuracy.

The position reconstruction unit 10C assigns, based on the direction of the movement of the ultrasound probe, weights to the motion vectors estimated by the first motion estimation unit and the second motion vector, combines the weighted first and second motion vectors into a combined motion vector, and assembles the images according to the combined motion vector, and thus generates the ultrasound diagnostic image of the subject.

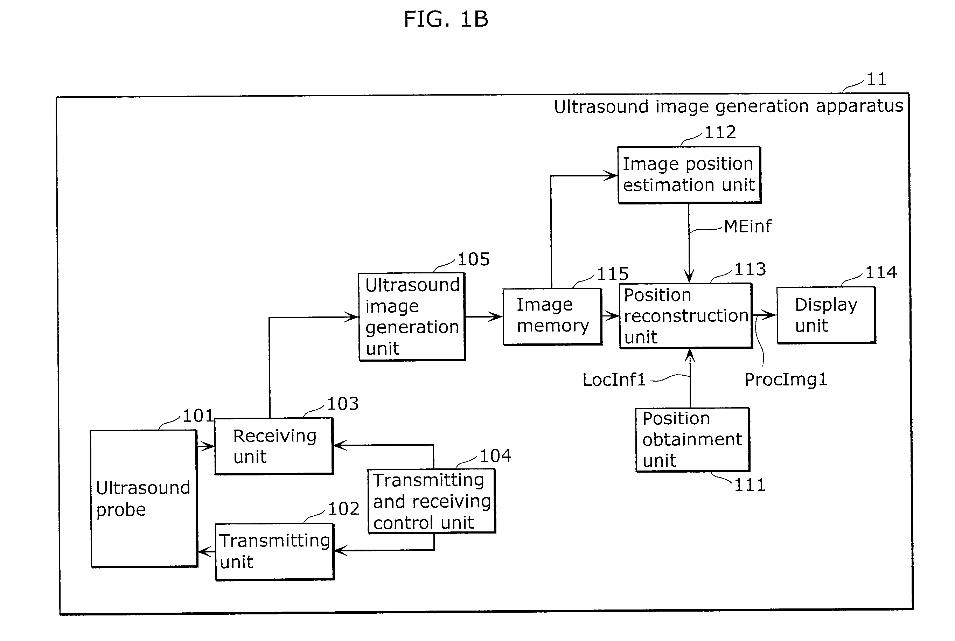

FIG. 1B is a configuration diagram of an ultrasound image generation apparatus 11 according to Embodiment 1 of the present invention.

As shown in FIG. 1B, the ultrasound image generation apparatus 11 includes: an ultrasound probe 101, a transmitting unit 102, a receiving unit 103, a transmitting and receiving control unit 104, an ultrasound image generation unit 105, an image memory 105, a position obtainment unit 111, an image position estimation unit 112, a position reconstruction unit 113, and a display unit 114. The operations of the functional blocks which transmit and receive the ultrasound signal, perform processing for generating a B-mode image or a Doppler image, and generate the ultrasound image are the same as the operations of the functional blocks of the conventional ultrasonic diagnostic apparatus 1501. Thus, those functional blocks are given the same reference numerals, and descriptions thereof are omitted. The image position estimation unit 112 corresponds to the first motion estimation unit 10A. The position obtainment unit 111 corresponds to the second motion estimation unit 10B.

Examples of the probes that can be used as the ultrasound probe 101 are: a linear type probe which includes at least a row of ultrasound transducers and obtains the two-dimensional image; an oscillating 3D type probe which obtains the three-dimensional image by sequentially generating two-dimensional images with a row of the ultrasound transducers that performs oscillation or parallel moving; and a matrix type probe which obtains three-dimensional image with the ultrasound transducers disposed in a two dimensional array. This embodiment describes, as an example, the case of using the linear type probe.

The position obtainment unit 111 obtains position information LocInf1 of the ultrasound probe 101, and inputs the position information LocInf1 to the position reconstruction unit 113. The position obtainment unit 111 and the position reconstruction unit 113 operate synchronously or operate according to the existing reference clock to match the obtainment timing of the position information and the obtainment timing of the ultrasound image. The position information includes six parameters that indicate the position in the three-dimensional space (corresponding to the coordinate values of x-axis, y-axis, and z-axis) and the orientation (which is information indicating the amount of rotation about each of the three axes, and is information that determines the orientation of the ultrasound probe in the three-dimensional space). As the position obtainment unit 101, various schemes can be used, such as: an optical scheme, such as a camera; a magnetic sensor; a gyro; an acceleration sensor; or GPS. In this embodiment, the case of using the camera is described as an example.

Based on the ultrasound images stored in an image memory 115, the image position estimation unit 112 estimates, based on the correlation between the images, a motion vector MEInf between the images, and input the motion vector MEInf to the position reconstruction unit 113.

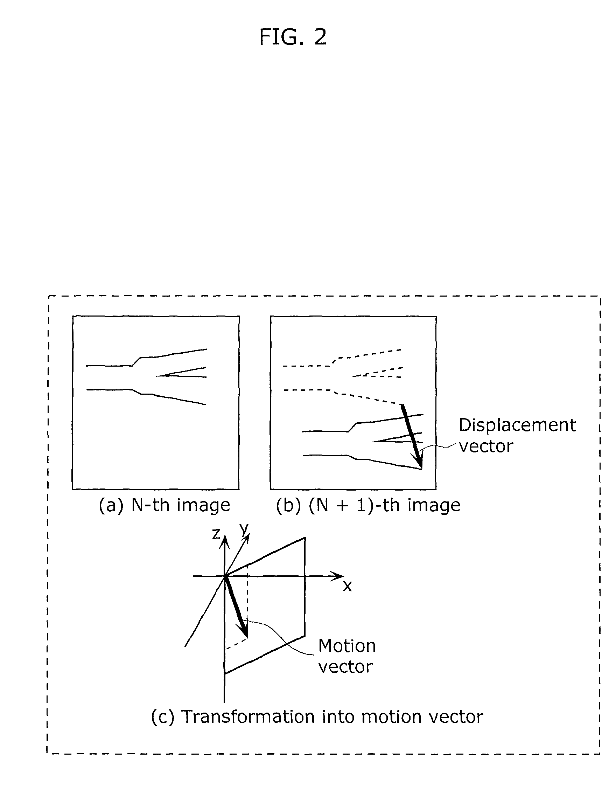

FIG. 2 is a descriptive diagram of a method for obtaining the motion vector by image processing. Shown in (a) in FIG. 2 is N-th obtained ultrasound image, and shown by the solid line in (b) in FIG. 2 is (N + 1)-th ultrasound image. The displacement which corresponds to the displacement vector shown in (b) in FIG. 2 exists between N-th image and (N + 1)-th image. The displacement is determined based on an evaluation value of the entire image or for each of the evaluation units which are obtained by dividing the image. For example, the sum of the differences in pixel values between the evaluation units may be assumed to be the evaluation value. The displacement may be determined by searching the evaluation unit which has the smallest evaluation value in (N + 1)-th image with respect to the evaluation unit in a particular position in N-th image, and calculating the displacement between the evaluation units. Furthermore, the displacement may be determined by an evaluation using an index, such as the correlation and an amount of mutual information of the pixel values between the evaluation units. Furthermore, a feature point may be detected based on an active contour model, such as the scale-invariant feature transform (SIFT) method or the SNAKES method, and the displacement vector may be determined using the average or the mean value of the displacement between a plurality of feature points. Furthermore, the center of gravity or the like of the contour may be calculated using the detected feature points, and the displacement of the center of gravity may be used as the displacement vector. For example, when capturing an image of a site where the contour is relatively clear, such as a blood vessel, the contour of the capturing target may be extracted in an image, and the region including the extracted contour may be used as the evaluation unit. Furthermore, the displacement vector may be obtained by using a point on the extracted contour as a feature point. The displacement vector shown in (b) in FIG. 2 is a vector on an image plane. Thus, the displacement vector is transformed into the vector in the three-dimensional space based on the position information of N-th image. Hereinafter, the vector which is in the three-dimensional space and indicates the displacement between two images is referred to as a motion vector. Shown in (c) in FIG. 2 is a result of transforming the displacement vector shown in (b) in FIG. 2 into the motion vector. The direction of movement and the movement distance of the ultrasound probe in the three-dimensional space correspond to the motion vector. It should be noted that the transformation into the motion vector may be performed by the position reconstruction unit 113. Furthermore, in the same manner, the motion vector can be calculated using the difference between position information of N-th image and (N + 1)-th image obtained by the position obtainment unit 111.

The position reconstruction unit 113 maps the ultrasound images to the three-dimensional space based on the position information determined by combining the position information LocInf1 and the motion vector MEinf in a weighted manner to construct the three-dimensional image, thereby generating a three-dimensional image ProcImg1 for display. Lastly, the display unit 114 displays the three-dimensional image ProcImg1 on an output device, such as a monitor. The ultrasound image generation apparatus 11 according to the present invention is characterized by the operations performed by the position obtainment unit 111, the image position estimation unit 112, and the position reconstruction unit 113. Thus, the following mainly describes the operations performed by these functional blocks, and the descriptions on the other functional blocks are omitted accordingly.

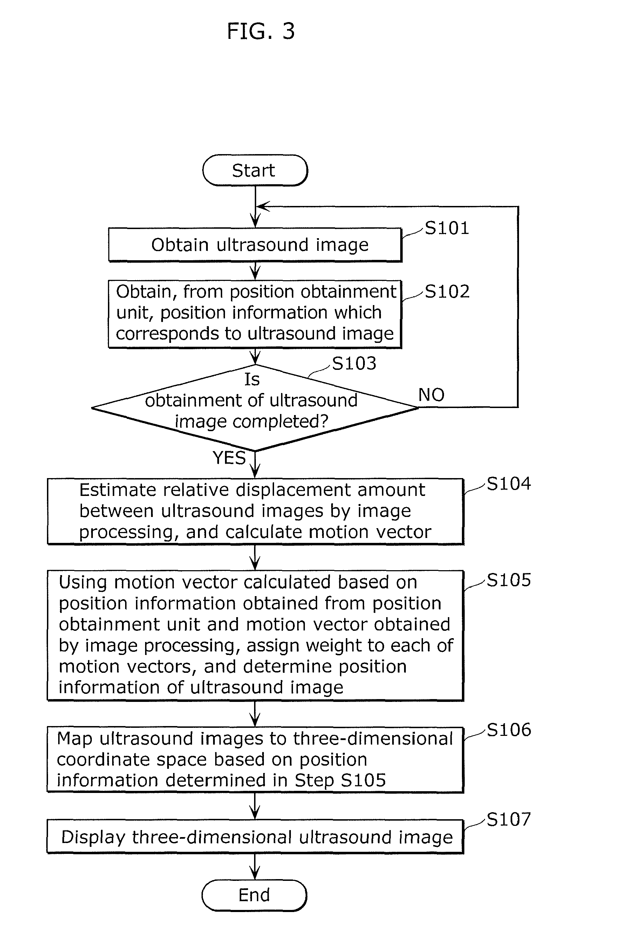

FIG. 3 is a flowchart of operations performed by the ultrasound image generation apparatus 11. As shown in FIG. 3, the ultrasound image generation apparatus 11 obtains the ultrasound image in Step S101. In Step S102, the ultrasound image generation apparatus 11 obtains, from the position obtainment unit 101, the position information which corresponds to the ultrasound image obtained in Step S101. In Step S103, the ultrasound image generation apparatus 11 determines whether or not the obtainment of the ultrasound image has been completed. The ultrasound image generation apparatus 11 repeats the processing in Step S101 and Step S102 until it is determined in Step 103 that the obtainment of the ultrasound image has been completed. In Step S104, the ultrasound image generation apparatus 11 estimates the relative amount of displacement between the ultrasound images by the image processing, and calculates the motion vector. In Step S105, based on the motion vector calculated based on the position information obtained from the position obtainment unit 111, and the motion vector obtained by the image processing, the ultrasound image generation apparatus 11 assigns weight to each of the motion vectors, and determines the position information of the ultrasound image. In Step S106, the ultrasound image generation apparatus 11 maps the ultrasound images to the three-dimensional coordinate space based on the position information determined in Step S105 to construct the three-dimensional image. In Step S107, the ultrasound image generation apparatus 11 displays the three-dimensional ultrasound image.

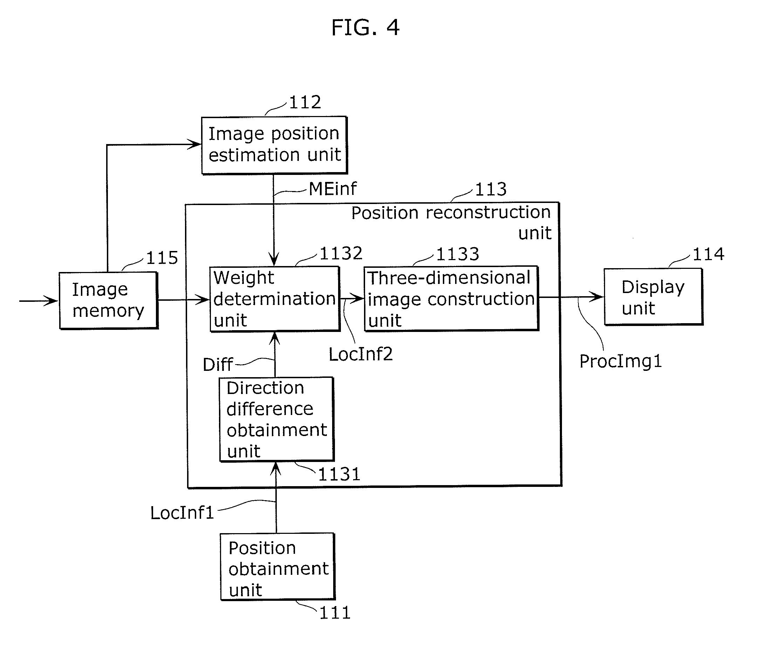

FIG. 4 is a block diagram showing a configuration of the position reconstruction unit 113. The position reconstruction unit 113 includes: a direction difference obtainment unit 1131, a weight determination unit 1132, and a three-dimensional image construction unit 1133.

The direction difference obtainment unit 1131 calculates a direction difference Diff between the motion vector obtained by the position obtainment unit 111 and the reference direction, that is to say, the angle formed by the motion vector and the reference direction. Here, the reference direction indicates a particular direction with respect to the position obtainment unit 111. The reference direction is, for example, a depth direction of the camera. Furthermore, the reference direction may be a normal direction of an imaging surface of the camera.

The weight determination unit 1132 determines a final motion vector by assigning, based on the direction difference Diff, a weight to the motion vector obtained by each of the position obtainment unit 111 and the image position estimation unit 112. In addition, the weight determination unit 1132 adds, to the position information of the image which is immediately preceding the target image to be processed, the motion vector to determine the position information of the target image to be processed, and input the determined position information to the three-dimensional image construction unit 1133 as position information LocInf2.

The three-dimensional image construction unit 1133 maps the ultrasound images to the three-dimensional space based on the position information LocInf2 to construct a three-dimensional image.

It should be noted that the position information itself can be added or subtracted when the direction included in the position information is represented by a format which allows addition and subtraction, such as quaternion. When the direction is represented by a format such as an Euler angle, the position information is transformed into a format which allows the addition and subtraction, and then addition or subtraction is performed on the position information. Furthermore, the position obtainment unit 111 obtains the position information relative to a reference position of the marker. Thus, when mapping the ultrasound images to the three-dimensional space, the three-dimensional image construction unit 1133 performs rotation and translation by taking into consideration the offset among the pixel position on the ultrasound image, the reference position of the marker, and the orientation of the marker.

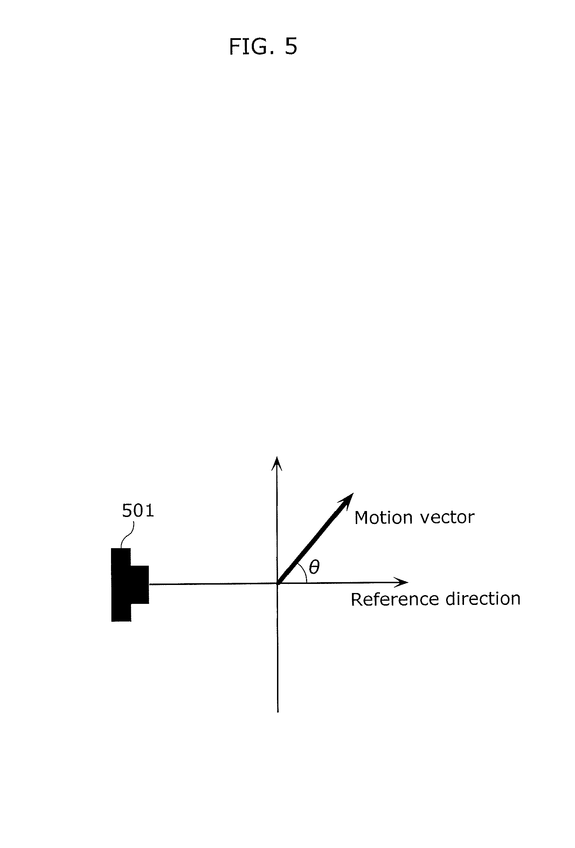

FIG. 5 is a descriptive diagram of an angle formed by the reference direction and the motion vector. Here, the depth direction of the camera in which a position resolution from the camera is low is set as the reference direction. Furthermore, when it is assumed that the angle formed by the reference direction and the motion vector is represented by $\theta$, the angle $\theta$ corresponds to the direction difference Diff. It should be noted that a motion vector obtained with the camera is used as the motion vector when determining the angle $\theta$. For example, the weight determination unit 1132 assigns, based on the angle $\theta$, weights to the motion vector obtained from the position obtainment unit 111 and the motion vector obtained by the image processing, using a method exemplified by (Expression 1).

[Math. 1]

$$ mv\_3 = (1 - \cos\theta) \times mv\_1 + \cos\theta \times mv\_2 \qquad \text{(Expression 1)} $$

Here, mv_3 represents the weighted motion vector, mv_1 represents the motion vector obtained from the position obtainment unit, and mv_2 represents the motion vector obtained by the image processing. The camera has low position resolution in the depth direction. Thus, when the motion vector is closer to the depth direction that is when the $\cos\theta$ is closer to 1, the weighting assigned to mv_1 is smaller.

It should be noted that the method for calculating the motion vector is not limited to the method shown in Expression (1). The function which monotonically increases or monotonically decreases when $\theta$ varies can also be used.

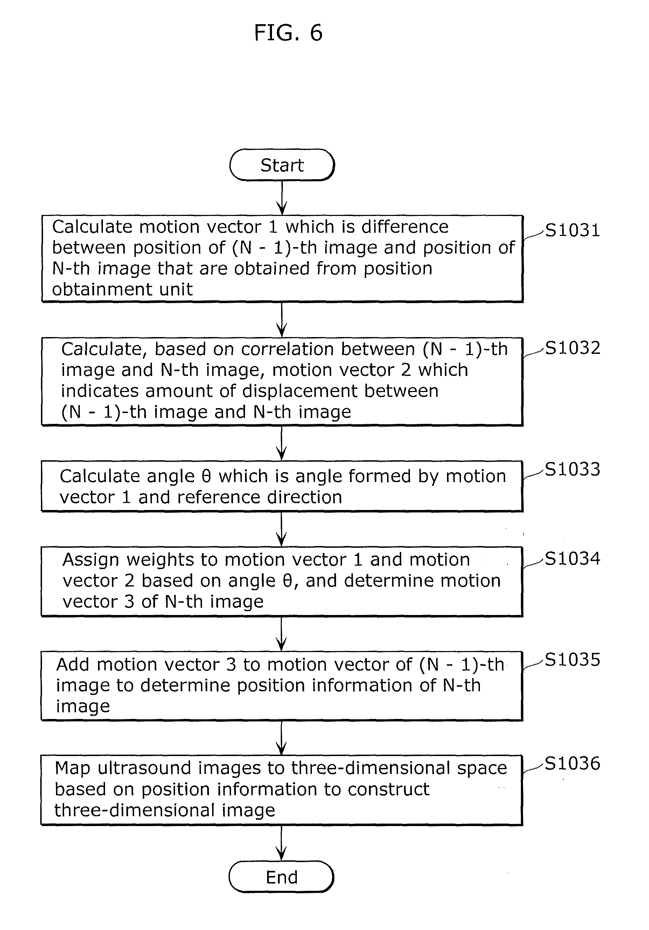

FIG. 6 is a flowchart of operations performed by the position reconstruction unit 113. In Step S1031, the position reconstruction unit 113 calculates a motion vector 1 which is a difference between the position of (N - 1)-th image and the position of N-th image that are obtained from the position obtainment unit 101. In Step S1032, the position reconstruction unit 113 calculates, based on the correlation between (N - 1)-th image and N-th image, a motion vector 2 which indicates amount of displacement between (N - 1)-th image and N-th image. In Step S1033, the position reconstruction unit 113 calculates angle $\theta$ which is the angle formed by the motion vector 1 and the reference direction. In Step S1034, the position reconstruction unit 113 assigns weights to the motion vector 1 and the motion vector 2 based on the angle $\theta$, and determines a motion vector 3 which is the final motion vector of N-th image. In Step S1035, the position reconstruction unit 113 adds the motion vector 3 to the position vector of (N - 1)-th image to determine the position of N-th image. Here, the position vector is a vector determined by the position and orientation of the image. It should be noted that the angle θ may be calculated based on the angle formed by the motion vector 3 of (N - 1)-th image and the reference direction. Furthermore, regarding the initial frame, the motion vector 1 is assumed to be the motion vector 3. In Step S1036, the position reconstruction unit 113 maps the ultrasound images to the three-dimensional space based on the position information to construct the three-dimensional image.

Next, another example of the method for assigning a weight based on angle θ is described. First, although the motion vector 1 and the motion vector 2 are weighted together based on the angle θ according to Expression (1), the motion vector may be resolved into direction components, and then weights may be assigned.

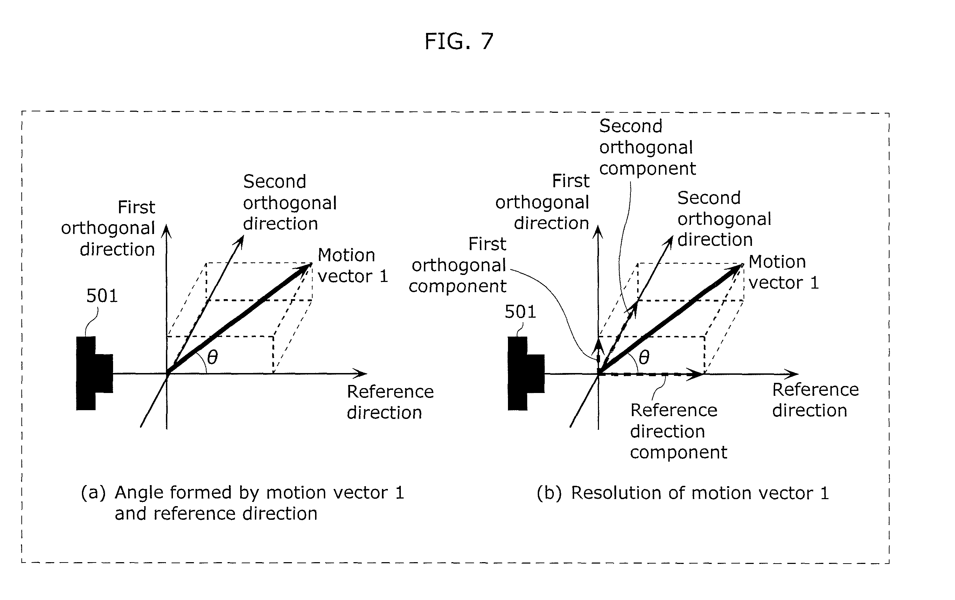

FIG. 7 shows an example of resolving the motion vector based on the reference direction. Shown in (a) in FIG. 7 is a relationship between the motion vector 1 and the reference direction. Shown in (b) in FIG. 7 is an example in which the motion vector 1 is resolved into three components, namely, a reference direction component, a first orthogonal component of the reference direction, and a second orthogonal component of the reference direction. The effect of the degradation of position resolution from the camera is significant on the reference direction component, and therefore the weighting assigned to the reference direction component is different from the weighting assigned to the other two directions. Expression (2) shows an example of the weighting.

[Math. 2]

$$mv\_3[ref] = (1 - \cos\theta) \times mv\_1[ref] + \cos\theta \times mv\_2[ref]$$
$$mv\_3[crs1] = (1 - \alpha) \times mv\_1[crs1] + \alpha \times mv\_2[crs1] \qquad \text{(Expression 2)}$$
$$mv\_3[crs2] = (1 - \beta) \times mv\_1[crs2] + \beta \times mv\_2[crs2]$$

Here, the definition of each of the vectors, which are mv_1, mv_2, and mv_3, is the same as the definition for Expression (1). [ref], [crs1], and [crs2] represents the reference direction component of the motion vector, the first orthogonal component of the reference direction, and the second orthogonal component of the reference direction, respectively. Furthermore, $\alpha$ represents the weight assigned to the first orthogonal component, and $\beta$ represents the weight assigned to the second orthogonal component. It should be noted that, by setting $\alpha$ or $\beta$ to 0, it is also possible to remove contribution from (no contribution is made by) the component in the corresponding direction.

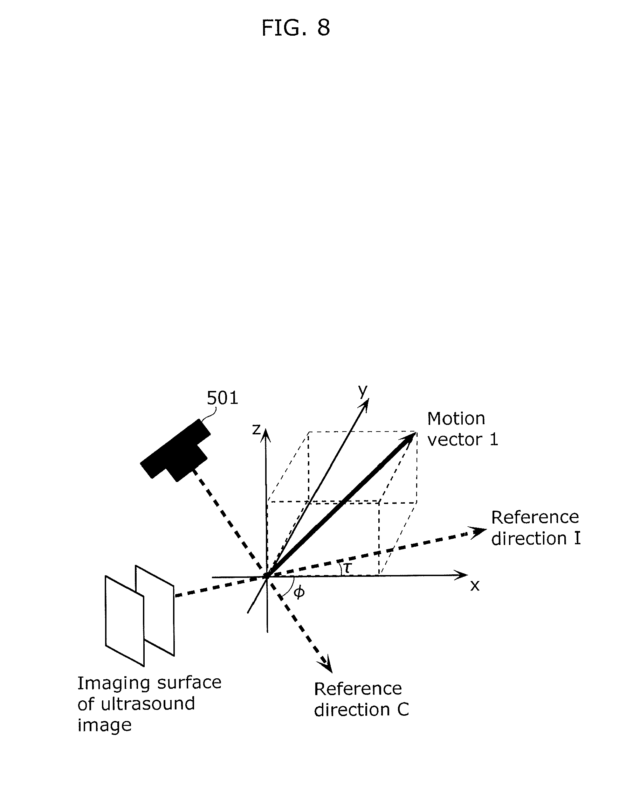

FIG. 8 is a diagram showing an example of resolving the motion vector into absolute coordinate space components. The absolute coordinate space is a three-dimensional space determined by pre-set three axes (x-axis, y-axis, and z-axis in FIG. 8) which are orthogonal to each other. The motion vector 1 is resolved into components in the three axial directions, namely, x, y, and z. At this time, the component in each axial direction is determined based on the angle formed by each axis and a reference direction C (the reference direction of the camera), and the angle formed by each axis and a reference direction I (the reference direction of image processing). Here, the reference direction I (the reference direction of image processing) is a direction in which the position resolution from the image processing is low, and indicates the normal direction of the imaging surface of the ultrasound image. Expression (3) shows an example of assigning a weight to the motion vector.

[Math. 3]

$$mv\_3[x] = c1 \times (1 - \cos\phi) \times mv\_1[x] + c2 \times (1 - \cos\tau) \times mv\_2[x] \qquad \text{(Expression 3)}$$

Here, $\varphi$ represents an angle formed by the reference direction C and x-axis, T represents an angle formed by the reference direction I and x-axis, and [x] represents a component in the x-axis direction of the motion vector. Each of c1 and c2 is a coefficient for normalizing the sum of the weights assigned to mv_1[x] and mv_2[x] to be one. It should be noted that y-axis direction and z-axis direction can be processed in a similar manner. This method is advantageous in that weighting can be flexibly assigned to the motion vector obtained with the camera and the motion vector obtained by the image processing.

It should be noted that ratio of contribution on mv_3[x] by each of mv_1[x], and mv_2[x] can be set by changing c1 and c2. For example, the contribution of each of mv_1[x] and mv_2[x] can be made equal by setting c1 and c2 to the same value. Furthermore, by setting c1 to a constant multiple of c2, it is possible to make the contribution of mv_1[x] to be a constant multiple of mv_2[x].

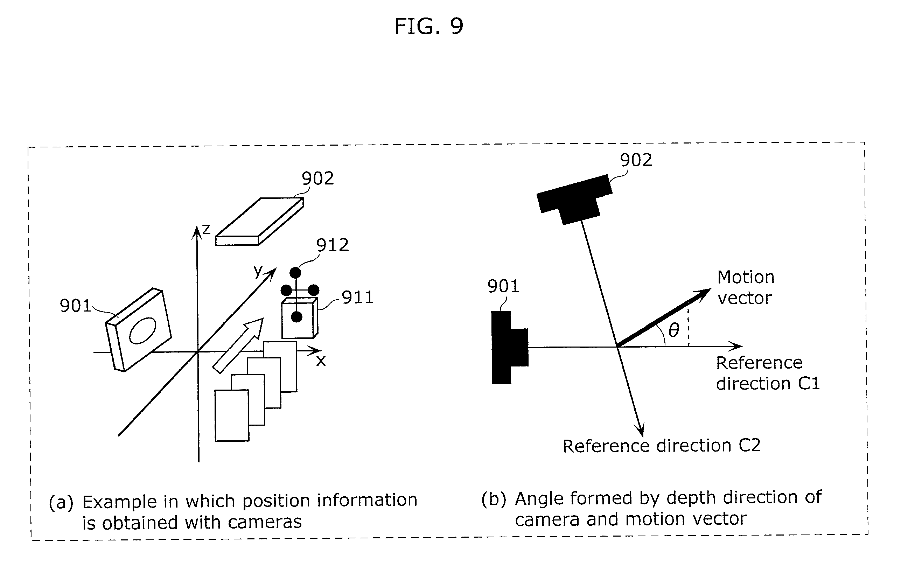

Furthermore, a plurality of cameras may be used as the position obtainment unit 111. FIG. 9 is a descriptive diagram of a method for obtaining position information with cameras. Shown in (a) in FIG. 9 is an example in which the position information is obtained using two cameras which are a camera 901 and a camera 902. At this time, it is preferable that the cameras be arranged spaced apart from each other facing different directions. The above arrangement makes it possible to (i) prevent the occurrence of the marker being obstructed, by an operator of the ultrasound probe or by the body of the subject, and not being captured with a camera and (ii) reduce the direction in which the position resolution is low, by arranging the cameras so that the cameras have different reference directions. Shown in (b) in FIG. 9 are the angle formed by a reference direction C1 (the reference direction of the camera 901) and the motion vector, and an angle formed by the reference direction C2 (the reference direction of the camera 2) and the motion vector. Here, as the motion vector used when determining the angle θ, it is possible to use the motion vector obtained with the camera 1, the motion vector obtained with the camera 2, or the motion vector 3 of an immediately preceding image.

The motion vector 3 that is the final motion vector is obtained by (i) determining the motion vector 1 by combining the motion vectors obtained by the cameras, (ii) assigning weights to the motion vector 1 and the motion vector 2 according to (Expression 1), and (iii) combining the weighted motion vector 1 and the weighted motion vector 2. Alternatively, instead of using a single vector which is obtained by combining the motion vectors obtained with the cameras, weighting may be assigned to the motion vectors obtained by the cameras and the motion vector 2. An example of the former is shown as (Expression 4).

[Math. 4]

$$mv\_1[ref1] = (1 - \cos\theta) \times mv\_11[ref] + \cos\theta \times mv\_12[ref]$$
$$mv\_1[crs1] = (1 - \alpha) \times mv\_11[crs1] + \alpha \times mv\_12[crs1]$$
$$mv\_1[crs2] = (1 - \beta) \times mv\_11[crs2] + \beta \times mv\_12[crs2]$$

(Expression 4)

Here, mv_1, mv_11, and mv_12 are respectively the motion vector 1 which is obtained by combining the motion vectors obtained with the cameras, the motion vector obtained with the camera 1, and the motion vector obtained with the camera 2. [ref], [crs1], and [crs2] represents the reference direction component of the motion vector, the first orthogonal component of the reference direction, and the second orthogonal component of the reference direction, respectively. Furthermore, α represents the weight assigned to the first orthogonal component, and β represents the weight assigned to the second orthogonal component. When determining mv_11, and mv_12, the position information obtained by the other of the cameras may be fed back. For example, the resolution from the camera 1 is low on the motion in x-axis direction. However, the position resolution in x-axis direction from the camera 1 is improved by using, when determining the position information with the camera 1, the amount of movement in x-axis direction obtained with the camera 2. It should be noted that although FIG. 9 describes the case in which the two cameras are used, extension is possible by using three or more cameras as well. Furthermore, a plurality of types of position sensors, such as a camera and a gyro, may be used in combination with each other.

In the above, the position obtainment unit 101 and the image processing are used together, and the motion vector is introduced to determine the position information because the image processing only provides a relative positional relationship. However, the position obtainment unit 101, such as the camera, can provide the absolute value of the position relative to an origin of the position obtainment unit 101. Therefore, when determining the position information based only on the position information obtained by a plurality of position obtainment units 101, the absolute value of the position obtained by each of the position obtainment units may be weighted, without using the motion vector.

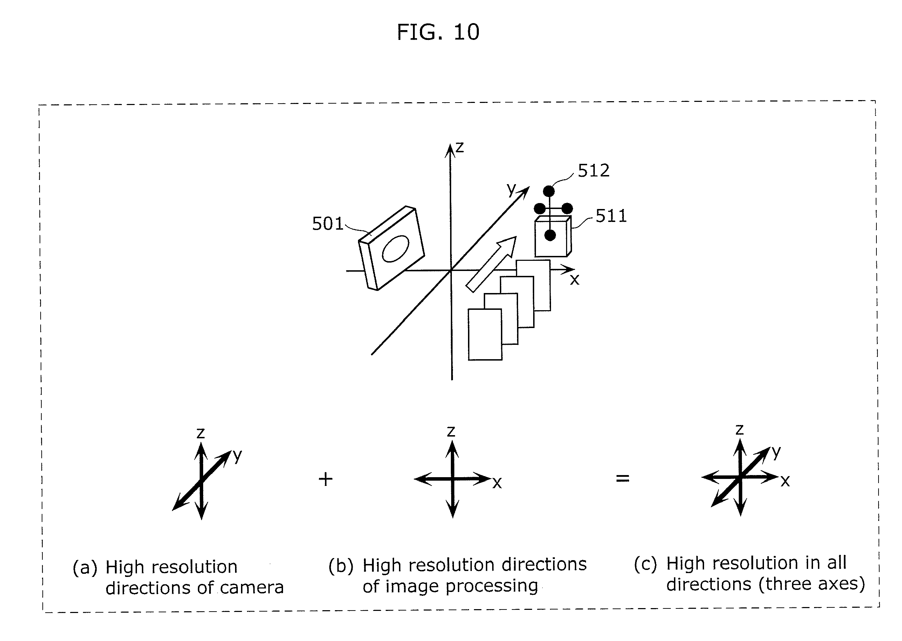

FIG. 10 is a diagram showing advantageous effects of the ultrasound image generation apparatus 11 according to Embodiment 1 of the present invention. In this example, the ultrasound probe moves in y-axis direction, and the reference direction of the camera is x-axis direction. Thus, with respect to the motion of the ultrasound probe, the camera can provide high resolution position information on yz-plane, and the image processing can provide high resolution position information on zx-plane. Therefore it is possible to obtain high resolution position information in all directions by using the camera and the image processing in combination with each other. In this manner, by combining a plurality of position information obtainment schemes, it is possible to obtain high resolution position information on the motion of the ultrasound probe in any direction.

As described above, with the ultrasound image generation apparatus according to an aspect of the present invention, a plurality of the motion vectors are estimated using the first and the second estimation methods having different direction dependency of the estimation accuracy, and the estimated motion vectors are combined. Thus, it is possible to obtain the motion vector of the ultrasound probe by combining the component of a direction in which the estimation accuracy from the first estimation method is relatively low with the component of a direction in which the estimation accuracy from the second estimation method is relatively high. At this time, it is possible to calculate the motion vector having high accuracy in all directions by assigning a lighter weight to the component having lower estimation accuracy and assigning a heavier weight to the component having higher estimation accuracy. Thus, it is possible to reduce direction dependency of accuracy in obtaining the position of the ultrasound probe.

Furthermore, it is possible to analyze the displacement of the two images and estimate the amount of movement of the ultrasound probe. This makes it possible to detect, with relatively high accuracy, the motion of the ultrasound probe on a plane including the cross-section of the subject which transmitted the ultrasound signals corresponding to the images.

Furthermore, it is possible to analyze the pixels which are included in the two images to calculate the amount of movement of the corresponding pixel or region, which is formed by a plurality of pixels, and estimate the amount of movement of the ultrasound probe based on the calculated amount of movement. This makes it possible to detect, with relatively high accuracy, the motion of the ultrasound probe on a plane including the cross-section of the subject corresponding to the images.

Furthermore, when generating the combined vector, a heavier weight is set to the component of a direction in which the estimation accuracy is relatively high from the estimation method, and then the weight is assigned to the estimated position vector. This makes it possible to increase the accuracy of the combined motion vector. Thus, it is possible to reduce direction dependency of accuracy in obtaining the position of the ultrasound probe.

Furthermore, when estimating the motion vector of the ultrasound probe by image analysis, a heavier weight is set to the direction component on a plane including the cross-section of the subject corresponding to the images, and then the combined position vector is generated. This makes it possible to increase the accuracy of the combined motion vector.

Furthermore, it is possible to estimate the motion vector of the ultrasound probe by the optical marker and the camera. This makes it possible to detect, with relatively high accuracy, the motion of the ultrasound probe on a plane parallel to the imaging surface of the camera.

Furthermore, when estimating the ultrasound probe by the optical marker and camera, a heavier weight is set to the direction component on a plane parallel to the imaging surface of the camera, and then the combined position vector is generated. This makes it possible to increase the accuracy of the combined motion vector.

Furthermore, it is possible to use both methods, namely, the position estimation of the ultrasound probe by the image processing, and the position estimation of the ultrasound probe by the optical marker and camera, and generate the combined position vector by combining the position vectors of the ultrasound probe which are estimated using the aforementioned methods.

(Embodiment 2)

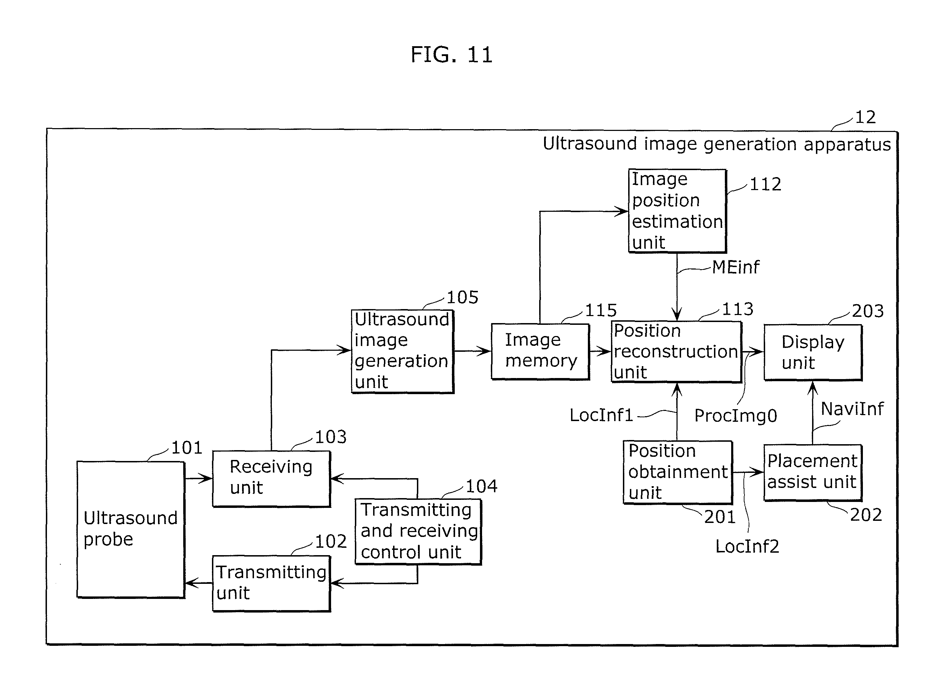

FIG. 11 is a block diagram showing configuration of an ultrasound image generation apparatus 12 according to Embodiment 2 of the present invention. The ultrasound image generation apparatus 12 includes: an ultrasound probe 101, a transmitting unit 102, a receiving unit 103, a transmitting and receiving control unit 104, an ultrasound image generation unit 105, an image memory 115, an image position estimation unit 112, a position reconstruction unit 113, a position obtainment unit 201, a placement assist unit 202, and a display unit 203. As described with reference to FIG. 10, the ultrasound image generation apparatus according to the present invention reduces the direction in which the position resolution is low, by complementing each other between a plurality of position obtainment units or between the position obtainment unit and the image processing. Compared to the ultrasound image generation apparatus 11, the ultrasound image generation apparatus 12 additionally includes the placement assist unit 202 which determines the placement position of the position obtainment unit 201, such as a camera. It should be noted that the same reference numerals are given to the functional blocks that are the same as the functional blocks included in the ultrasound image generation apparatus 11, and the descriptions thereof are omitted.

The placement assist unit 202 determines the placement target position of the position obtainment unit 201 based on the position information LocInf2, which indicates the initial position, obtained from the position obtainment unit 201, and generates assist information NaviInf which is for placing the position obtainment unit to the placement target position, and input the assist information NaviInf to the display unit 203. The display unit 203 displays the assist information NaviInf on a display device.

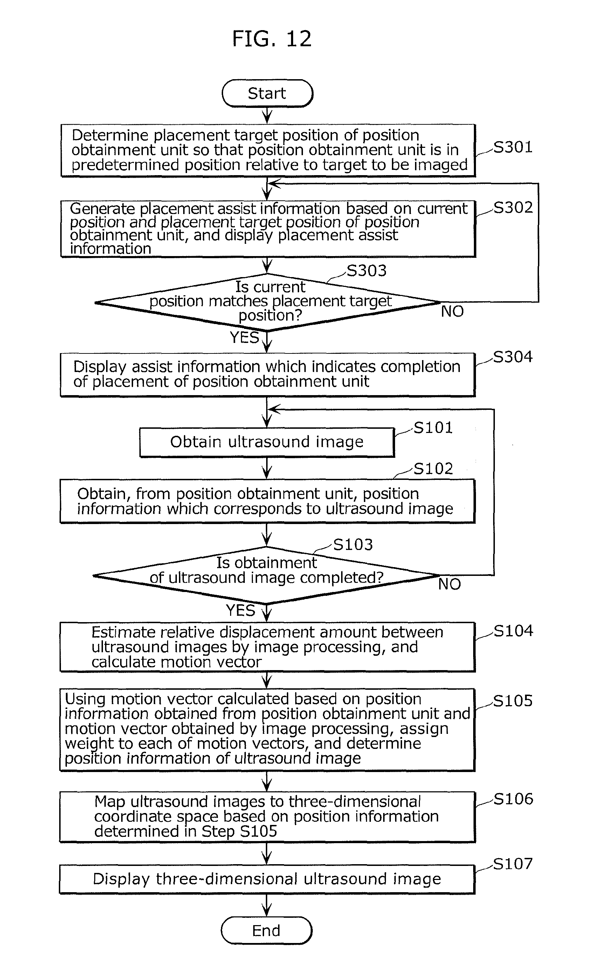

FIG. 12 is a flowchart showing operations performed by the ultrasound image generation apparatus 12. First, in Step S301, the ultrasound image generation apparatus 12 obtains the current position of the position obtainment unit 201, and determines the placement target position of the position obtainment unit 201 so that the position obtainment unit 201 is in a predetermined position relative to the target to be imaged. The following describes the processing performed when a camera is used as the position obtainment unit 201.

First, in the current position, the ultrasound image generation apparatus 12 captures with the camera an image of the calibration marker placed near the site of interest of the subject to be imaged, and calculates the position of the camera relative to the calibration marker. Alternatively, the position of the camera relative to the target position to be imaged may be determined by scanning, with the ultrasound probe to which the marker is attached, the site of interest to be imaged and obtaining the position of the marker. Next, a method for determining the placement target position is described. The position resolution in the normal direction of the imaging surface of the ultrasound probe is low in the case of image processing. Thus, the camera is placed so that the difference between (i) the direction in which the position resolution of the camera is high and (ii) the normal direction of the imaging surface is equal to or less than a threshold value. The normal direction of the imaging surface can be obtained, by placing the calibration marker having a predetermined positional relationship relative to the normal direction, and capturing the calibration marker with the camera. Furthermore, the normal direction of the imaging surface can also be obtained by scanning the site to be imaged, and capturing the movement direction of the ultrasound probe with the camera. Furthermore, the camera has low position resolution in the depth direction. Thus, when a plurality of cameras is used, the cameras are placed so that the depth direction of a camera lies in the direction in which the other one of the cameras has high position resolution.

For example, when two cameras are used, the two cameras are placed so that the angle formed by the depth directions of the cameras be close to 90 degrees (i.e. approximately orthogonal to each other). When the angle formed by the depth directions of the cameras is 90 degrees and the motion vector of the ultrasound probe matches the depth direction of one of the cameras, the position resolution from the one of the camera is significantly lowered. However, the motion vector of the ultrasound probe does not match the depth direction of the other of the cameras at the same time. Therefore, the resolution of position obtainment by the two cameras is maintained at a certain level or greater. It should be noted that, the angle formed by the depth directions of the cameras may be set in a predetermined range close to 90 degrees. For example, the angle may be set in a range from 80 degrees to 100 degrees or may be set in other ranges. It should be noted that the distance between the camera and the site of interest to be imaged is determined so that the range of movement of the ultrasound probe is in the field of view of the camera.

In Step S302, the ultrasound image generation apparatus 12 generates, based on the current position and the placement target position of the position obtainment unit 201, the assist information which is for guiding the position obtainment unit 201 to the placement target position, and displays the assist information. Subsequently, a user moves the position obtainment unit 201 according to the assist information so that the position of the position obtainment unit 201 matches the placement target position.

In Step S303, the ultrasound image generation apparatus 12 determines whether or not the difference in the position and the orientation between the current position and the placement target position is equal to or less than the threshold value, and repeats the processes in Step S301 and Step S302 until the difference becomes equal to or less than the threshold value.

When it is determined in Step S303 that the difference in position and orientation between the current position and the placement target position is equal to or less than the threshold value, the ultrasound image generation apparatus 12 displays, in Step S304, assist information which indicates the completion of the placement of the position obtainment unit 201. The subsequent operations are the same as operations performed by the ultrasound image generation apparatus 11.

It should be noted that a plurality of the position obtainment units 201 may be provided. When the position obtainment units 201 are used, the ultrasound image generation apparatus 12 determines, in S302, the current position and the placement target position for each of the position obtainment units 201. Furthermore, the position obtainment unit 201 does not necessarily have to be placed by a user. The position obtainment unit 201 may be attached to a movable device, such as an electrically-driven stage or a robot arm, to be automatically moved to the placement target position. In the above, the placement of the position obtainment unit 201 is performed only once before capturing an ultrasound image. However, in practice, the placement target position changes when the travel direction changes. Therefore, when the position obtainment unit 201 can be automatically moved, the ultrasound image generation apparatus 12 may calculate the placement target position according to the travel direction, and move the position obtainment unit 201 to the calculated placement target position.

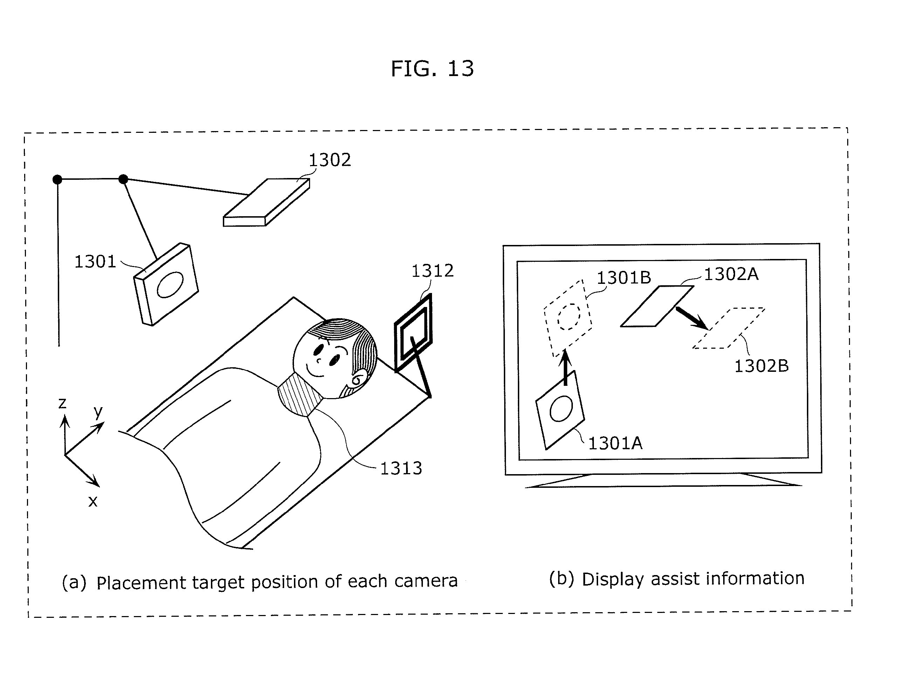

FIG. 13 is a descriptive diagram regarding the placement of the camera and the assist information on the placement of the camera. Shown in (a) in FIG. 13 is an example of the placement target position of the camera when capturing an image of a carotid artery positioned near the neck of a human. A camera 1 (1301) and a camera 2 (1302) are placed so that a neck 1313, which is a site of interest to be imaged, is in their field of views, and their depth directions cross each other to form an angle close to a right angle. A calibration marker 1312 is used to obtain the current position, and is set so that the relative position of the neck 1313 and the calibration marker 1312 is constant. With this, it is possible to obtain the relative position of the neck 1313 and the cameras (1301 and 1302).

Shown in (b) in FIG. 13 is an example display of the assist information. In FIG. 13, the solid lines indicate the current position (1301A) of the camera 1 and the current position (1302A) of the camera 2. Furthermore, the broken lines indicate the placement target position (1301B) of the camera 1 and the placement target position (1302B) of the camera 2. The arrows in the drawing indicate the movement directions for guiding each of the cameras to their respective placement target positions, and the user moves the cameras in the directions indicated by the arrows. It should be noted that, compared to movement in z-direction shown in (a) in FIG. 13, the ultrasound probe is more often moved in the direction of x-axis or y-axis, when capturing the neck. Thus, when one camera is used, the movement in the x-axis direction and the movement in the y-axis direction in which the position resolution obtained by image processing is low can be obtained with high resolution by placing the camera at the position to look down the neck as shown with the camera 2 (1302) in the drawing.

The following describes a variation of the embodiments.

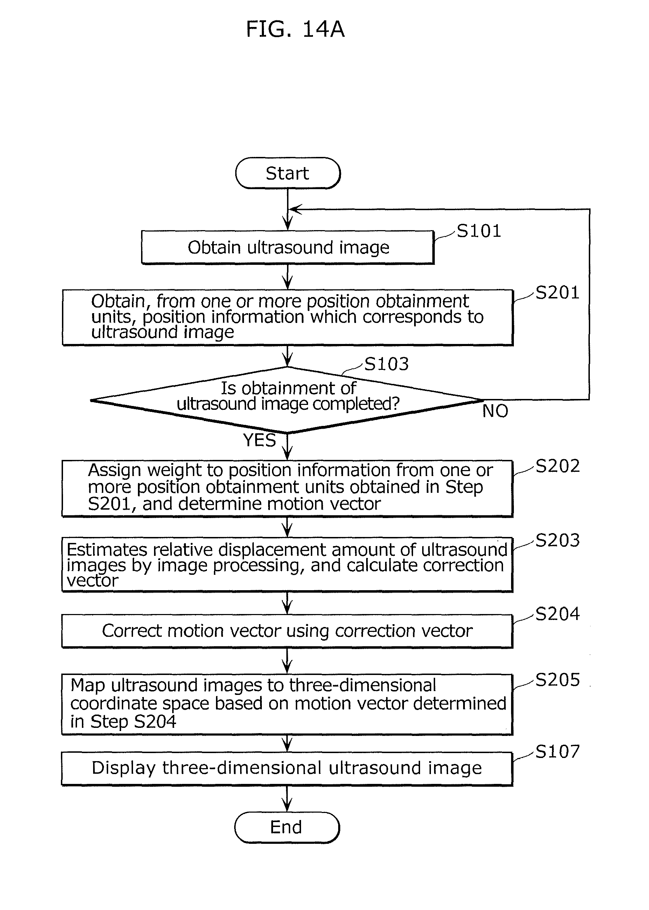

FIG. 14A is a flowchart showing operations performed by a variation of the ultrasound image generation apparatus 11 according to Embodiment 1. The ultrasound image generation apparatus assigns a weight to a motion vector obtained from one or more position obtainment units to determine the motion vector of the ultrasound image once, and then corrects the motion vector by image processing. The steps the same as the steps in FIG. 3 are given the same reference numerals, and the descriptions thereof are omitted.

First, in the steps up to Step S103, the ultrasound image generation apparatus completes the obtainment of the ultrasound image, and the obtainment of the position information from the position obtainment unit. In Step S201, the ultrasound image generation apparatus obtains the position information from the one or more position obtainment units.

In Step S202, the ultrasound image generation apparatus weights the position information from the one or more position obtainment units obtained in Step S201, and determines the motion vector.

In Step S203, the ultrasound image generation apparatus estimates a relative displacement amount between ultrasound images by the image processing, and transforms the estimated amount of displacement into the vector in the three-dimensional space, and thus calculates the correction vector. The amount of displacement between (N - 1)-th image and N-th image may be estimated after moving N-th image based on the motion vector of N-th image which is determined in Step S202. With this, it is possible to roughly estimate the amount of displacement using the motion vector obtained from the position obtainment unit, and then perform high accuracy estimation by the image processing. This makes it possible to reduce the amount of processing, and increase the robustness in position estimation. Furthermore, when setting the correction vector, continuity of the amount of movement among a plurality of consecutive images may be taken into consideration so that the motion vector changes smoothly. For example, when the motion vector obtained from the position obtainment unit fluctuates due to, for example, an unsteady movement of a hand or an error of the position obtainment unit, smoothing the change in motion vector by the image processing makes it possible to reduce the fluctuation and approximate the precise position. In addition, to estimate the amount of displacement, instead of using the correlation between the images, the motion vector obtained by the position obtainment unit may be smoothed by interpolation processing, such as spline interpolation, or by filter processing. The difference between the motion vector before and after the smoothing may be used as the correction vector. It should be noted that, without using the motion vector, the smoothing processing may be performed on the position information obtained from the position obtainment unit, and may obtain, as the motion vector, the difference between the position information before and after the smoothing.

In Step S204, the ultrasound image generation apparatus corrects the motion vector by adding the correction vector to the motion vector.

In Step S205, the ultrasound image generation apparatus constructs the three-dimensional image by mapping the ultrasound images to the three-dimensional space based on the corrected motion vector, and displays the three-dimensional image in Step S107.

Furthermore, the position reconstruction 103 may perform the following operations to reduce effects of the error in obtaining the position information or unsteady movement of a hand. First, reliability of the position information obtained by the position obtainment unit or the image processing is set so as not to use the position information of low reliability. For example, when the position information obtained by one camera and the image processing is used and the reliability of the position information obtained by the image processing is equal to or less than the threshold value, only the position information obtained with the camera is used. Alternatively, when the position information obtained by two cameras and the image processing is used and the reliability of the position information from one of the cameras is low, the position information from the other of the cameras and the image processing is used. Furthermore, for example, when the position information having reliability equal to or greater than a threshold value does not exist, use of the corresponding ultrasound image may be avoided in constructing the three-dimensional image. The amount of change in the motion vectors between the consecutive images can be used as the reliability of the position obtainment unit. For example, when the difference in the absolute values or the directions between the motion vector of (N - 1)-th image and the motion vector of N-th image exceeds a threshold value, the position information of N-th image is invalidated. In the image processing, in addition to a similar criterion, for example, when searching the evaluation unit having the lowest evaluation value in estimating the amount of displacement, the position information may be invalidated if the smallest evaluation value exceeds a threshold value. In addition, the errors on the position information may accumulate, when the position information of the preceding image is used in determining the final position information based on a motion vector 3 or the motion vector 3. Thus, the accumulation of errors may be reduced by resetting the position information at predetermined scan time intervals or for every obtainment of a predetermined number of images so that the position information of the image is determined based only on the position information of the image to be processed.

Furthermore, the above described an example in which the position obtainment unit is the camera, and a weight is assigned to the position information based on the difference in direction between the reference direction and the direction of the motion vector. However, the criterion for assigning a weight is not limited to the difference in directions.

For example, the weight may be assigned based on a distance between a magnetic transmitter in a magnetic sensor and the probe or, when the resolution is different depending on the direction of rotation, such as a gyro, the weight may be assigned according to the direction of rotation.

It should be noted that although the apparatus which constructs the three-dimensional image based on the position information obtained with high position resolution has been described thus far in the above, the application is not limited to the construction of the three-dimensional image. For example, the above can be applied to align an ultrasound image and a two-dimensional image or a three-dimensional image captured by a modal, such as CT and MRI, other than the ultrasound; or to align position of the ultrasound images which are obtained on different dates, such as at the time of periodic diagnoses. Furthermore, the position obtainment unit and the image processing need not necessarily be used together. In particular, when a plurality of position obtainment units is used, the position information may be determined based only on the position obtainment units.

It should be noted that the following makes it possible to improve the accuracy in position obtainment performed by the position obtainment unit 111 and the image position estimation unit 112.

Trade-off relationship exists between the improvement in accuracy of position obtainment and the increase in the frequency of position obtainment performed by the position obtainment unit 111 and the image position estimation unit 112. The accuracy of information can be improved by performing, in the position obtainment unit 111 and the image position estimation unit 112, high accuracy or robust position information determination processing on the obtained information, for example, by an iterative operation. However, a predetermined amount of time is needed to perform the high accuracy or robust position information determination processing, and thus the frequency of position obtainment is decreased.

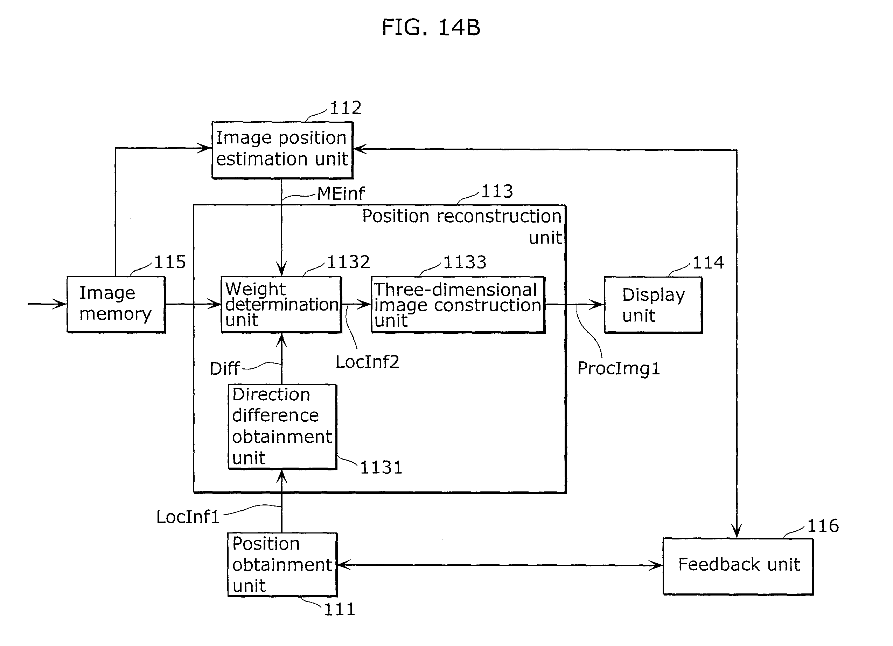

In view of this, the frequency of the position obtainment performed by the position obtainment unit 111 and the image position estimation unit 112 is changed according to the accuracy of position obtained by the position obtainment unit 111 and the image position estimation unit 112. Specifically, frequency of the position obtainment is controlled by feeding back the difference between the position information obtained by the position obtainment unit 111 and the position information obtained by the image position estimation unit 112. Each of the position obtainment unit 111 and the image position estimation unit 112 has an operation mode in which the accuracy or the robustness is low and the position obtainment frequency is high (high frequency mode), and an operation mode in which the accuracy or the robustness is high and the position obtainment frequency is low (high accuracy mode), and these operation modes are switched according to the feedback information. This makes it possible to improve the accuracy in or frequency of position obtainment performed by the position obtainment unit 111 and the image position estimation unit 112 according to the status of position obtainment. FIG. 14B shows an example of the feedback control.

FIG. 14B shows a configuration diagram of an example of the ultrasound image generation apparatus including a feedback unit. As shown in FIG. 14B, in this example, the ultrasound image generation apparatus includes a feedback unit 116. The operations performed by the other functional blocks are the same as the operations performed by the functional blocks included in the ultrasonic diagnostic apparatus 11. Thus, the same functional blocks are given the same reference numerals, and descriptions thereof are omitted.

Specifically, the feedback unit 116 obtains the position information obtained by the position obtainment unit 111 and the image position estimation unit 112. Then, the feedback unit 116 calculates the difference between the position information, and switches the position obtainment unit 111 and the image position estimation unit 112 to the high accuracy mode, when the difference is greater than a threshold value. Furthermore, when the difference between the position information is smaller than the threshold value, the feedback unit 116 switches the position obtainment unit 111 and the image position estimation unit 112 to the high frequency mode. This makes it possible to improve the accuracy in or frequency of position obtainment performed by the position obtainment unit 111 and the image position estimation unit 112 according to the status of position obtainment.

As described above, with the ultrasound image generation apparatus according to an aspect of the present invention, an operator can set the direction of the estimation method so that the direction in which the estimation accuracy from an estimation method is low matches the direction in which the estimation accuracy is high from another estimation method, in order to reduce the direction dependency of accuracy in estimating the combined position vector. Thus, it is possible to reduce direction dependency of accuracy in obtaining the position of the ultrasound probe.

(Embodiment 3)

The processing described in each of the above embodiments can be easily implemented on an independent computer system, by recording, on a recording medium such as a flexible disk, a program for realizing the ultrasound image generation method described in the above embodiments.

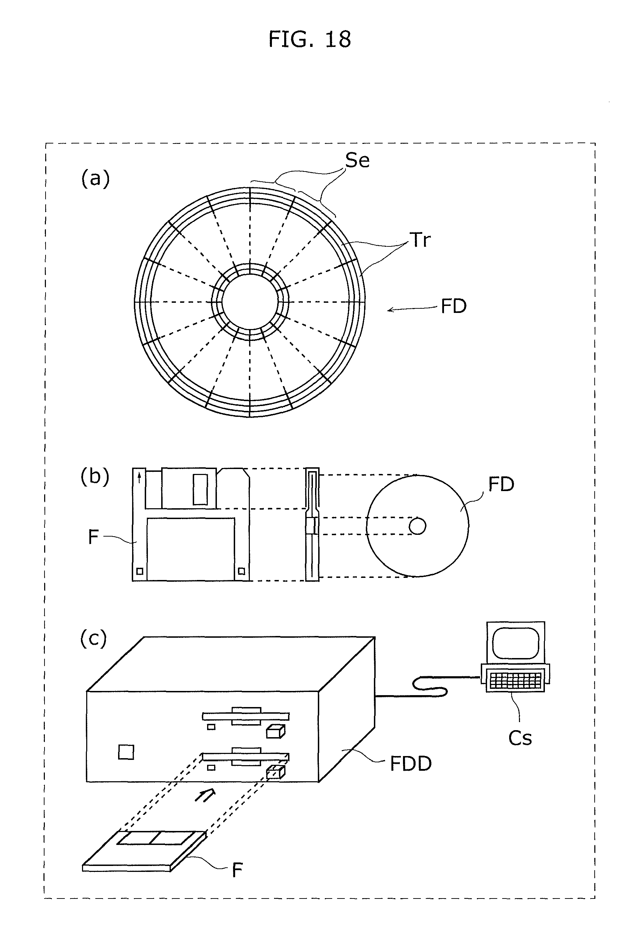

FIG. 18 is diagrams explaining the case in which the ultrasound image generation method according to the above-described embodiments is implemented by a computer system using a program recorded on a recording medium, such as a flexible disk.

Shown in (b) in FIG. 18 is a front appearance of the flexible disk, a cross section of the flexible disk, and the flexible disk. Shown in (a) in FIG. 18 is an example of a physical format of the flexible disk as a recording medium body. A flexible disk FD is contained in a case F, and a plurality of tracks Tr are concentrically formed on a surface of the flexible disk FD from outer to inner peripheries. Each track is divided into 16 sectors Se in an angular direction. This being so, in the flexible disk storing the program, the program is recorded in an area allocated on the flexible disk FD.

Shown in (c) in FIG. 18 is a structure of recording and reproducing the program on the flexible disk FD. In the case of recording, on the flexible disk FD, the above program which realizes the image processing method, the above program is written from a computer system Cs via a flexible disk drive. In the case of implementing, on the computer system, the image processing method which realizes the image processing method by the program recorded on the flexible disk, the program is read from the flexible disk and transferred to the computer system via the flexible disk drive.

Although the above describes an example of using the flexible disk as the recording medium, an optical disc may equally be used. Moreover, the recording medium is not limited to such, and any recording medium such as an IC card, a ROM cassette, and the like is applicable so long as the program can be recorded.

It should be noted that each of the blocks, such as an ultrasound image generation unit 105, a position obtainment unit 111, an image position estimation unit 112, a position reconstruction unit 113, and an image memory 115 shown in FIG. 1B, is typically implemented as an LSI (Large Scale Integration) which is an integrated circuit. These blocks may be individually configured as single chips or may be configured so that a part or all of the blocks are included in a single chip.

Although the name used here is LSI, it may be referred to as any of an IC (integrated circuit), a system LSI, a super LSI, or an ultra LSI, depending on the difference in degree of integration.

Moreover, ways to achieve integration are not limited to an LSI, and may be implemented by a special circuit or a general-purpose processor. For example, a special circuit for graphics processing, such as Graphic Processing Unit (GPU) can be used. A Field Programmable Gate Array (FPGA) which allows programming after LSI manufacturing or a reconfigurable processor which allows reconfiguration of the connections and settings of the circuit cells inside the LSI may also be used.

Furthermore, when an integrated circuit technology that replaces an LSI emerges from advancement of semiconductor technologies or other derivative technologies, such a technique can naturally be used for the functional block integration. The adaptation of biotechnology or the like is one of the possibilities.

In the exemplary embodiments above, each of the constituent elements may be implemented as a piece of special hardware or implemented by executing a software program appropriate for each of the constituent elements. The constituent elements may also be implemented by a program execution unit such as a CPU or a processor which reads and executes a software program recorded on a recording medium, such as a hard disk or a semiconductor memory. Here, the ultrasound image generation apparatus and the like in the above-described embodiments are implemented by executing a software program below.

Specifically, the program causes the computer to execute an ultrasound image generation method for generating an ultrasound diagnostic image from a plurality of ultrasound signals received from a subject while an ultrasound probe is being moved, the ultrasound image generation method includes: estimating, as a first motion vector, a motion vector indicating a movement of the ultrasound probe using a first estimation method, the motion vector indicating the movement between a plurality of images of the respective ultrasound signals; estimating, as a second motion vector, a motion vector indicating a movement of the ultrasound probe using a second estimation method which is different from the first estimation method in direction dependency of estimation accuracy, the motion vector indicating the movement between the images of the respective ultrasound signals; and (i) assigning, based on a direction of the first motion vector or a direction of the second motion vector, weights to the first motion vector estimated in the estimating of the first motion vector and the second motion vector estimated in the estimating of the second motion vector, (ii) combining the weighted first and second motion vectors into a combined motion vector, and (iii) constructing the ultrasound diagnostic image of the subject using the combined motion vector and the images.

Furthermore, the program causes the computer to execute, in the estimating of the first motion vector, estimation of a motion vector of the ultrasound probe, the estimation being performed by calculating, based on image displacement between two images among the images, the motion vector indicating a movement made from when one of the two images is obtained till when the other of the two images is obtained.

The ultrasound image generation apparatus according to one of or more of the aspects of the present invention has been described based on the embodiments. However, the present invention is not limited to these embodiments. Various modifications of the exemplary embodiment as well as embodiments resulting from any combinations of constituent elements of different exemplary embodiments that may be conceived by those skilled in the art may be included within the scope according to one of or more of the aspects of the present invention as long as these do not depart from the essence of the present invention.

[Industrial Applicability]

An ultrasound image generation apparatus and a method according to the present invention determine, when constructing a three-dimensional image using position information and ultrasound images obtained by scanning in an arbitrary direction the target to be imaged, the final position information by assigning weights to position information obtained from a plurality of position obtainment units. Thus, it is possible to obtain high accuracy position information relative to the movement in the arbitrary direction, and construct a high accuracy three-dimensional image. Therefore, the ultrasound image generation apparatus and the method make it possible to observe, with high accuracy, the three-dimensional shape of the site to be diagnosed, and thus can improve the diagnosis accuracy, and are highly applicable in particular to medical diagnostic appliance industry.

[Reference Signs List]

| | |
|---|---|
| 10, 11, 12 | Ultrasound image generation apparatus |
| 10A | First motion estimation unit |
| 10B | Second motion estimation unit |
| 10C, 113, 1508 | Position reconstruction unit |
| 101 | Ultrasound probe |
| 102 | Transmitting unit |
| 103 | Receiving unit |
| 104 | Transmitting and receiving control unit |
| 105 | Ultrasound image generation unit |
| 111, 201, 1507 | Position obtainment unit |
| 112 | Image position estimation unit |
| 114, 203, 1509 | Display unit |
| 115, 1506 | Image memory |
| 1131 | Direction difference obtainment unit |
| 1132 | Weight determination unit |
| 1133 | Three-dimensional image construction unit |

**Claims**

1.  An ultrasound image generation apparatus which generates an ultrasound diagnostic image from a plurality of ultrasound signals received from a subject while an ultrasound probe is being moved, the ultrasound image generation apparatus comprising:

    a first motion estimation unit configured to estimate, as a first motion vector, a motion vector indicating a movement of the ultrasound probe using a first estimation method, the motion vector indicating the movement between a plurality of images of the respective ultrasound signals;
    a second motion estimation unit configured to estimate, as a second motion vector, a motion vector indicating a movement of the ultrasound probe using a second estimation method which is different from the first estimation method in direction dependency of estimation accuracy, the motion vector indicating the movement between the images of the respective ultrasound signals; and
    a position reconstruction unit configured to (i) assign, based on a direction of the first motion vector or a direction of the second motion vector, weights to the first motion vector estimated by the first motion estimation unit and the second motion vector estimated by the second motion estimation unit, (ii) combine the weighted first and second motion vectors into a combined motion vector, and (iii) construct the ultrasound diagnostic image of the subject using the combined motion vector and the images.

2.  The ultrasound image generation apparatus according to Claim 1,
    wherein the first motion estimation unit is configured to estimate a motion vector of the ultrasound probe by calculating, based on image displacement between two images among the images, the motion vector indicating a movement made from when one of the two images is obtained till when the other of the two images is obtained.

3.  The ultrasound image generation apparatus according to Claim 2,

wherein the first motion estimation unit is configured to estimate the motion vector of the ultrasound probe from when the one of the two images among the images is obtained till when the other of the two images is obtained, by detecting an amount of the image displacement between the two images based on a correlation between pixel values of pixels included in the two images.

4. The ultrasound image generation apparatus according to any one of Claims 1 to 3, wherein, the position reconstruction unit is configured to (i) calculate the combined motion vector by assigning a heavier weight to the first motion vector or the second motion vector when an angle formed by a movement direction of the ultrasound probe and a corresponding one of reference directions is greater, and (ii) construct the ultrasound diagnostic image using the combined motion vector, the corresponding one of the reference directions being a direction used as a reference by the first motion estimation unit or the second motion estimation unit when the estimation is made.

5. The ultrasound image generation apparatus according to Claim 4, wherein the position reconstruction unit is configured to (i) calculate the combined motion vector by assigning a heavier weight to the motion vector estimated by the first motion estimation unit when an angle formed by a movement direction of the ultrasound probe and the reference direction used by the first motion estimation unit is greater, and (ii) construct the ultrasound diagnostic image using the combined motion vector, the reference direction being a direction in which estimation accuracy is low.

6. The ultrasound image generation apparatus according to Claim 1, wherein at least one of the first motion estimation unit and the second motion estimation unit is configured to capture, with a camera, an image of an optical marker attached to the ultrasound probe, and calculate a position and an angle of the ultrasound probe based on a position or a shape of the optical marker in the captured image.

7. The ultrasound image generation apparatus according to Claim 6, wherein the position reconstruction unit is configured to (i) calculate the combined motion vector by assigning a heavier weight to the motion vector estimated by the optical motion estimation unit when an angle formed by a movement direction of the ultrasound probe and a normal direction of an imaging surface of the camera is greater, and (ii) assemble the images using the combined motion vector, the normal direction being a reference direction used by the optical motion estimation unit.

8. The ultrasound image generation apparatus according to Claim 1, wherein the first motion estimation unit is configured to estimate a motion vector of the ultrasound probe by calculating, based on image displacement between two images among the images, the motion vector indicating a movement made from when one of the two images is obtained till when the other of the two images is obtained, and the second motion estimation unit is configured to estimate a motion vector of the ultrasound probe by capturing, with a camera, an image of an optical marker attached to the ultrasound probe, and calculating a position and an angle of the ultrasound probe based on a position or a shape of the optical marker in the captured image.

9. The ultrasound image generation apparatus according to any one of Claims 1 to 8, further comprising a placement assist unit configured to present information which prompts an operator to perform an operation for changing at least one of (i) a reference direction used by the first motion estimation unit and (ii) a reference direction used by the second motion estimation unit to allow the reference directions to be substantially orthogonal to each other.

10. An ultrasound image generation method for generating an ultrasound diagnostic image from a plurality of ultrasound signals received from a subject while an ultrasound probe is being moved, the ultrasound image generation method comprising:

estimating, as a first motion vector, a motion vector indicating a movement of the ultrasound probe using a first estimation method, the motion vector indicating the movement between a plurality of images of the respective ultrasound signals;
estimating, as a second motion vector, a motion vector indicating a movement of the ultrasound probe using a second estimation method which is different from the first estimation method in direction dependency of estimation accuracy, the motion vector indicating the movement between the images of the respective ultrasound signals; and

(i) assigning, based on a direction of the first motion vector or a direction of the second motion vector, weights to the first motion vector estimated in the estimating of the first motion vector and the second motion vector estimated in the estimating of the second motion vector, (ii) combining the weighted first and second motion vectors into a combined motion vector, and (iii) constructing the ultrasound diagnostic image of the subject using the combined motion vector and the images.

11. The ultrasound image generation method according to Claim 10,
wherein in the estimating of the first motion vector, a motion vector of the ultrasound probe is estimated by calculating, based on image displacement between two images among the images, the motion vector indicating a movement made from when one of the two images is obtained till when the other of the two images is obtained.

12. A program for causing a computer to execute the method according to Claim 10.

13. An integrated circuit which generates an ultrasound diagnostic image from a plurality of ultrasound signals received from a subject while an ultrasound probe is being moved, the integrated circuit comprising:

a first motion estimation unit configured to estimate, as a first motion vector, a motion vector indicating a movement of the ultrasound probe using a first estimation method, the motion vector indicating the movement between a plurality of images of the respective ultrasound signals;
a second motion estimation unit configured to estimate, as a second motion vector, a motion vector indicating a movement of the ultrasound probe using a second estimation method which is different from the first estimation method in direction dependency of estimation accuracy, the motion vector indicating the movement between the images of the respective ultrasound signals; and
a position reconstruction unit configured to (i) assign, based on a direction of the first motion vector or a direction of the second motion vector, weights to the first motion vector estimated by the first motion estimation unit and the second motion vector estimated by the second motion estimation unit, (ii) combine the weighted first and second motion vectors into a combined motion vector, and (iii) construct the ultrasound diagnostic image of the subject using the combined motion vector and the images.

EP 2 716 230 A1

# FIG. 1A

Ultrasound image
generation apparatus — 10

First motion
estimation unit — 10A

Position
reconstruction unit — 10C

Second motion
estimation unit — 10B

21

FIG. 1B

Ultrasound image generation apparatus 11

Image position estimation unit 112

MEinf

Ultrasound image generation unit 105

Image memory 115

Position reconstruction unit 113

Display unit 114

LocInf1        ProcImg1

Position obtainment unit 111

Receiving unit 103

Ultrasound probe 101

Transmitting and receiving control unit 104

Transmitting unit 102

EP 2 716 230 A1

FIG. 2

(a) N-th image        (b) (N + 1)-th image

Displacement vector

Motion vector

(c) Transformation into motion vector

# FIG. 3

Start

Obtain ultrasound image — S101

Obtain, from position obtainment unit, position information which corresponds to ultrasound image — S102

S103
Is obtainment of ultrasound image completed?
NO

YES

Estimate relative displacement amount between ultrasound images by image processing, and calculate motion vector — S104

Using motion vector calculated based on position information obtained from position obtainment unit and motion vector obtained by image processing, assign weight to each of motion vectors, and determine position information of ultrasound image — S105

Map ultrasound images to three-dimensional coordinate space based on position information determined in Step S105 — S106

Display three-dimensional ultrasound image — S107

End

FIG. 4

EP 2 716 230 A1

FIG. 5

501

Motion vector

θ

Reference direction

FIG. 6

Start

Calculate motion vector 1 which is difference between position of (N - 1)-th image and position of N-th image that are obtained from position obtainment unit ⟋S1031

Calculate, based on correlation between (N - 1)-th image and N-th image, motion vector 2 which indicates amount of displacement between (N - 1)-th image and N-th image ⟋S1032

Calculate angle θ which is angle formed by motion vector 1 and reference direction ⟋S1033

Assign weights to motion vector 1 and motion vector 2 based on angle θ, and determine motion vector 3 of N-th image ⟋S1034

Add motion vector 3 to motion vector of (N - 1)-th image to determine position information of N-th image ⟋S1035

Map ultrasound images to three-dimensional space based on position information to construct three-dimensional image ⟋S1036

End

# FIG. 7

(a) Angle formed by motion vector 1
    and reference direction

(b) Resolution of motion vector 1

EP 2 716 230 A1

FIG. 8

501

y

z

Motion
vector 1

Reference
direction I

τ

x

φ

Reference
direction C

Imaging surface
of ultrasound
image

FIG. 9

(a) Example in which position information is obtained with cameras

(b) Angle formed by depth direction of camera and motion vector

EP 2 716 230 A1

FIG. 10

(a) High resolution directions of camera

(b) High resolution directions of image processing

(c) High resolution in all directions (three axes)

FIG. 11

Ultrasound image generation apparatus 12

- Image position estimation unit 112 — MEinf
- Ultrasound image generation unit 105
- Image memory 115
- Position reconstruction unit 113
- Display unit 203
- LocInf1 — ProcImg0 — NaviInf
- Position obtainment unit 201 — LocInf2
- Placement assist unit 202
- Ultrasound probe 101
- Receiving unit 103
- Transmitting and receiving control unit 104
- Transmitting unit 102

EP 2 716 230 A1

32

# FIG. 12

Start

Determine placement target position of position obtainment unit so that position obtainment unit is in predetermined position relative to target to be imaged — S301

Generate placement assist information based on current position and placement target position of position obtainment unit, and display placement assist information — S302

S303
Is current position matches placement target position?  NO

YES

Display assist information which indicates completion of placement of position obtainment unit — S304

Obtain ultrasound image — S101

Obtain, from position obtainment unit, position information which corresponds to ultrasound image — S102

S103
Is obtainment of ultrasound image completed?  NO

YES

Estimate relative displacement amount between ultrasound images by image processing, and calculate motion vector — S104

Using motion vector calculated based on position information obtained from position obtainment unit and motion vector obtained by image processing, assign weight to each of motion vectors, and determine position information of ultrasound image — S105

Map ultrasound images to three-dimensional coordinate space based on position information determined in Step S105 — S106

Display three-dimensional ultrasound image — S107

End

FIG. 13

(a) Placement target position of each camera

(b) Display assist information

# FIG. 14A

```
                        ( Start )
                            │
                            ▼
        ┌───────────────────────────────────┐  S101
        │      Obtain ultrasound image      │
        └───────────────────────────────────┘
                            │
                            ▼
        ┌───────────────────────────────────┐  S201
        │ Obtain, from one or more position │
        │ obtainment units, position        │
        │ information which corresponds to  │
        │ ultrasound image                  │
        └───────────────────────────────────┘
                            │
                            ▼          S103
                    ╱─────────────────╲
                   ╱   Is obtainment of ╲        NO
                  ◇ ultrasound image completed? ◇──────┐
                   ╲                   ╱                │
                    ╲─────────────────╱                │
                        YES │                          │
                            ▼                          │
        ┌───────────────────────────────────┐  S202    │
        │ Assign weight to position         │          │
        │ information from one or more      │          │
        │ position obtainment units obtained│          │
        │ in Step S201, and determine       │          │
        │ motion vector                     │          │
        └───────────────────────────────────┘          │
                            │                          │
                            ▼                          │
        ┌───────────────────────────────────┐  S203    │
        │ Estimates relative displacement   │          │
        │ amount of ultrasound images by    │          │
        │ image processing, and calculate   │          │
        │ correction vector                 │          │
        └───────────────────────────────────┘
                            │
                            ▼
        ┌───────────────────────────────────┐  S204
        │ Correct motion vector using       │
        │ correction vector                 │
        └───────────────────────────────────┘
                            │
                            ▼
        ┌───────────────────────────────────┐  S205
        │ Map ultrasound images to three-   │
        │ dimensional coordinate space based│
        │ on motion vector determined in    │
        │ Step S204                         │
        └───────────────────────────────────┘
                            │
                            ▼
        ┌───────────────────────────────────┐  S107
        │ Display three-dimensional         │
        │ ultrasound image                  │
        └───────────────────────────────────┘
                            │
                            ▼
                        ( End )
```

FIG. 14B

FIG. 15

Ultrasonic diagnostic apparatus
1501

Ultrasound probe
101

Transmitting unit
102

Transmitting and receiving control unit
104

Receiving unit
103

Ultrasound image generation unit
105

Image memory
1506

Position obtainment unit
1507

Position reconstruction unit
1508

Display unit
1509

LocInf0

ProcImg0

# FIG. 16

```
                    ┌─────────────┐
                    │    Start     │
                    └──────┬───────┘
                           │
                           ▼
      ┌────────────────────────────────────┐  ╱S1601
      │      Obtain ultrasound image        │
      └────────────────────┬───────────────┘
                           │
                           ▼
   ┌───────────────────────────────────────┐  ╱S1602
   │ Obtain, from position obtainment unit, │
   │ position information which corresponds  │
   │ to ultrasound image                    │
   └───────────────────────┬────────────────┘
                           │
                           ▼        ╱S1603
              ◇◇◇◇◇◇◇◇◇◇◇◇◇◇◇◇◇◇◇◇◇◇◇◇◇
             ◇  Is obtainment of ultrasound  ◇──── NO ──┐
              ◇  image completed?          ◇            │
                    ◇◇◇◇◇◇◇◇◇◇◇◇                        │
                    YES │                               │
                        ▼                               │
   ┌────────────────────────────────────────┐  ╱S1604   │
   │ Map ultrasound images to three-dimensional │       │
   │ coordinate space based on corresponding position│  │
   │ information                             │           │
   └────────────────────┬────────────────────┘          │
                        │                                │
                        ▼                                │
   ┌────────────────────────────────────────┐  ╱S1605    │
   │ Display three-dimensional ultrasound image │        │
   └────────────────────┬────────────────────┘          │
                        │                                │
                        ▼                                │
                 ┌─────────────┐                         │
                 │    End       │                         │
                 └─────────────┘                         │
```

FIG. 17

(a) Position obtainment with camera

(b) Position obtainment by image processing

FIG. 18

(a)

Se

Tr

FD

(b)

F

FD

(c)

FDD

Cs

F

<div align="center">

**INTERNATIONAL SEARCH REPORT**

</div>

| | International application No. |
|---|---|
| | PCT/JP2012/003523 |

**A.  CLASSIFICATION OF SUBJECT MATTER**
*A61B8/00*(2006.01)i

According to International Patent Classification (IPC) or to both national classification and IPC

**B.   FIELDS SEARCHED**

Minimum documentation searched (classification system followed by classification symbols)
A61B8/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

**C.   DOCUMENTS CONSIDERED TO BE RELEVANT**

| Category* | Citation of document, with indication, where appropriate, of the relevant passages | Relevant to claim No. |
|---|---|---|
| A | JP 2008-125692 A  (Aloka Co., Ltd.),<br>05 June 2008 (05.06.2008),<br>entire text; all drawings<br>(Family: none) | 1-13 |
| A | JP 2005-103328 A  (Hitachi, Ltd.),<br>21 April 2005 (21.04.2005),<br>entire text; all drawings<br>(Family: none) | 1-13 |
| A | JP 2002-102223 A  (Mitani Sangyo Co., Ltd.),<br>09 April 2002 (09.04.2002),<br>entire text; all drawings<br>(Family: none) | 1-13 |

[X] Further documents are listed in the continuation of Box C.　　　[ ] See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 18 June, 2012 (18.06.12) | 26 June, 2012 (26.06.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

## INTERNATIONAL SEARCH REPORT

| International application No. |
|---|
| PCT/JP2012/003523 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2001-157677 A  (Hitachi Medical Corp.),<br>12 June 2001 (12.06.2001),<br>entire text; all drawings<br>(Family: none) | 1-13 |
| A | JP 11-313822 A  (General Electric Co.),<br>16 November 1999 (16.11.1999),<br>entire text; all drawings<br>& US 6012458 A          & US 6106470 A<br>& DE 19910771 A         & DE 19910771 A1<br>& IL 128866 A | 1-13 |
| A | JP 8-332187 A  (Aloka Co., Ltd.),<br>17 December 1996 (17.12.1996),<br>entire text; all drawings<br>(Family: none) | 1-13 |

Form PCT/ISA/210 (continuation of second sheet) (July 2009)

**EP 2 716 230 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010075503 A